# EUROPEAN PATENT APPLICATION

(11) **EP 1 905 825 A1**
(43) Date of publication of application: **02.04.2008**
(21) Application number: 06450139.8
(22) Date of filing: 29.09.2006
(51) Int. Cl.: C12N 9/10, C12N 15/82, A01H 5/00

(54) **Galactosyltransferase**

(71) Applicant: Greenovation Biotech GmbH, 79111 Freiburg i.Br (DE)
(72) Inventor: Launhardt, Heike, 79104 Freiburg (DE); Stemmer, Christian, 79104 Freiburg (DE); Jost, Wolfgang, 79111 Freiburg (DE); Gorr, Gilbert, 79112 Freiburg (DE); Reski, Ralf, 79254 Oberried (DE); Rensing, Stefan, 79194 Gundelfingen (DE)
(74) Representative: Alge, Daniel

(57) **Abstract**

The invention discloses DNA molecules encoding galactosyltransferases, recombinant host cells, tissues or organisms comprising dysfunctional galactosyltransferase gene(s), recombinant host cells, tissues or organisms comprising an introduced functional galactosyltransferase gene, methods for the production of proteins therewith, methods for the production of galactosyltransferase and vectors and uses thereof.

## Description

The present invention relates to polynucleotides coding for glycosyltransferases. Moreover, the present invention relates to partial polynucleotides thereof as well as to vectors comprising these polynucleotides in purposes of expression or gene disruption thereof, recombinant host cells, tissue or organisms transfected with the polynucleotides or parts thereof or DNA derived therefrom, as well as glycoproteins produced in these host cells, tissue or organisms. Furthermore, the present invention relates to the use of the expression product thereof *in vitro* as well as *in vivo.*

In the past, heterologous proteins have been produced using a variety of transformed cell systems, such as those derived from bacteria, fungi, such as yeasts, insect, plant or mammalian cell lines.

Proteins produced in prokaryotic organisms may not be post-translationally modified in a similar manner to that of eukaryotic proteins produced in eukaryotic systems, e.g. they may not be glycosylated with appropriate sugars at particular amino acid residues, such as aspartic acid (N) residues (N-linked glycosylation). Furthermore, folding of bacterially-produced eukaryotic proteins may be inappropriate due to, for example, the inability of the bacterium to form cysteine disulfide bridges. Moreover, bacterially-produced recombinant proteins frequently aggregate and accumulate as insoluble inclusion bodies.

Eukaryotic cell systems are better suited for the production of glycosylated proteins found in various eukaryotic organisms, such as humans, since such cell systems may effect post-translational modifications, such as N-glycosylation of produced proteins. However, a problem encountered in eukaryotic cell systems which have been transformed with heterologous genes suitable for the production of protein sequences destined for use, for example, as pharmaceuticals, is that the glycosylation pattern on such proteins often acquires a native pattern, that is, of the eukaryotic cell system in which the protein has been produced: glycosylated proteins are produced that comprise non-animal glycosylation patterns and these in turn may be immunogenic and/or allergenic if applied in animals, including humans. In plants this limitation has been overcome by the elimination of the plant-specific sugar residues 1,2-xylose and α1,3-fucose which in plants are generally linked to the core structure of N-glycans (Lerouge et al. 1998 Plant Mol. Biol. 38, 31-48; Rayon et al. 1998 J. Experimental Bot. 49, 1463-1472). In case of *Arabidopsis thaliana* (Strasser et al. 2004 FEBS Lett. 561, 132-136) and in case of the bryophyte *Physcomitrella patens* (EP1431394 ; Koprivova et al. 2004 Plant Biotechnol. J. 2, 517-523) mutants were generated showing N-glycan patterns completely lacking core α1,3-fucose and 1,2-xylose residues. Surprisingly, despite the modification of the pattern of the complex-type N-glycans no morphological alterations or changes in viability were observed in these mutants.

Apart from the addition of the two plant-specific residues described above the steps of glycoprotein maturation in the ER and in the cis-Golgi are identical in plants and mammals up to the action of GlcNAc-transferase I, GlcNAc-transferase II and Golgi a-mannosidase (Lerouge et al. 1998 Plant Mol. Biol. 38, 31-48). Further N-glycan elongation is carried out in a different manner in the two kingdoms. While in mammals the terminal GlcNAc residues are immediately shielded by the action of β1,4- (or, seldom, by β1,3-)- galactosyltransferase - with the notable exception of IgG where this step only occurs partially - elongation in plants is exclusively by β1,3-galactosylation but only a very small part of the glycans appear to undergo this modification as can be deduced from the relative abundance of various structural types . The galactose-residues in mammals may be capped by sialic acid and only quite rarely substituted by fucose. Again, plants are different, as they are devoid of sialylation and in case that a terminal 1,3-linked galactose residue was attached they essentially always fucosylate the penultimate GlcNAc residue, thereby forming a Lewis a (LeA) determinant. Apparently, the β1,3-galactosyltransferase is the limiting enzyme whereas most plant cells contain sufficient activity of α1,4-Fuc-transferase to make sure that each Gal containing antenna is fucosylated. The LeA structure is a human blood group determinant. It is rare as such in healthy adults but as sialyl-Lewis a (sLeA) it is notoriously found in malignant tissues such as colon cancer.

Anyway, LeA containing glycoproteins are rarely isolated from plants and in case of *Physcomitrella* they present an amount of only up to five percent of totally solulable glyco-proteins - irrespective if isolated from wild type plants or isolated from the glyco-engineered mutants lacking core fucose and xylose (Koprivova et al. 2003 Plant Biol. 5, 582-591; Koprivova et al. 2004 Plant Biotechnol. J. 2, 517-523).

Whereas some investigations were performed regarding the α1,4-fucosyltransferase which is involved in the generation of Lewis a type glycan structures in plants (Joly et al. 2002 J. Experimental Bot. 53, 1429-1436; Bakker et al. 2001 FEBS Lett. 507, 307-312) there is no information available regarding a specific β1,3 galactosyltransferase which is involved in the elongation of N-glycan structures in plants.

In eukaryotes β1,3-galactosyltransferases show a broad spectrum of acceptor specifities as well as distinct patterns of tissue expression (Hennet 2002 Cell. Mol. Life Sci. 59, 1081-1095; Amado et al. 1998 J. Biol. Chem. 21, 12770-12778). Among the different members of the β1,3-galactosyltransferase family of humans for β1,3-galactosyltransferase 2 it has been shown *in vitro* that this enzyme was active toward the transfer of galactose residues to GlcNAcβand egg ovalbumin -representing complex-type N-glycan structures as acceptor substrates (Amado et al. 1998 J. Biol. Chem. 21, 12770-12778).

According to the existence of a family of homologous β1,3-galactosyltransferases in humans data base analysis revealed that in different plant species e.g. *Arabidopsis thaliana* and *Oryza sativa* similar large gene families of β1,3-galactosyltransferase genes exist. None of the members of these β1,3-galactosyltransferase genes is described as coding for an enzyme which comprise the ability to transfer galactose from UDP-galactose to acceptor substrates with terminal non-reducing GlcNAc residues e.g. to non-reducing terminal residues of the complex-type N-glycans neither *in vitro* nor *in vivo.*

It is an object of the present invention to identify and to clone and to sequence one or more genes - including non-coding corresponding genomic sequences - which code for plant β1,3galactosyltransferases, and to prepare vectors comprising the genes, DNA fragments thereof or an altered DNA or a DNA derived thereof or DNA comprising deletions thereof. It is a further objective to generate host cells, tissue or organisms comprising one or more of these vectors, to produce glycoproteins completely lacking Lewis a type N-glycan structures. It is a further objective to generate host cells, tissue or organisms comprising one or more of these vectors, to produce glycoproteins with improved Lewis a type N-glycan structures. It is a further objective to provide nucleotide sequences encoding membrane domains for targeting enzymes to the late Golgi cisternae.

Accordingly, the present invention provides i) a DNA molecule comprising a sequence according to SEQ ID NO: 1 having an open reading frame from base pair 513 to base pair 2417 or having at least 50% identity with the above-mentioned sequence or comprising a sequence which has degenerated to the above DNA sequence due to the genetic code, the sequence coding for a plant protein which has β1,3-galactosyltransferase activity or is complementary thereto, ii) a DNA molecule comprising a sequence according to SEQ ID NO: 2 having an open reading frame from base pair 1 to base pair 1902 or having at least 50% identity with the above-mentioned sequence or comprising a sequence which has degenerated to the above DNA sequence due to the genetic code, the sequence coding for a plant protein which has β1,3-galactosyltransferase activity or is complementary thereto, iii) a DNA molecule comprising a sequence according to SEQ ID NO: 3 representing the genomic DNA structure from base pair 1 to base pair 6187 including intron sequences and exon sequences corresponding to SEQ ID NO: 1 allowing generation of knockout constructs with genomic sequences, iv) a DNA molecule comprising a sequence according to SEQ ID NO: 4 representing the genomic DNA structure from base pair 1 to base pair 4087 including intron sequences and exon sequences corresponding to SEQ ID NO: 2 allowing generation of knockout constructs with genomic sequences.

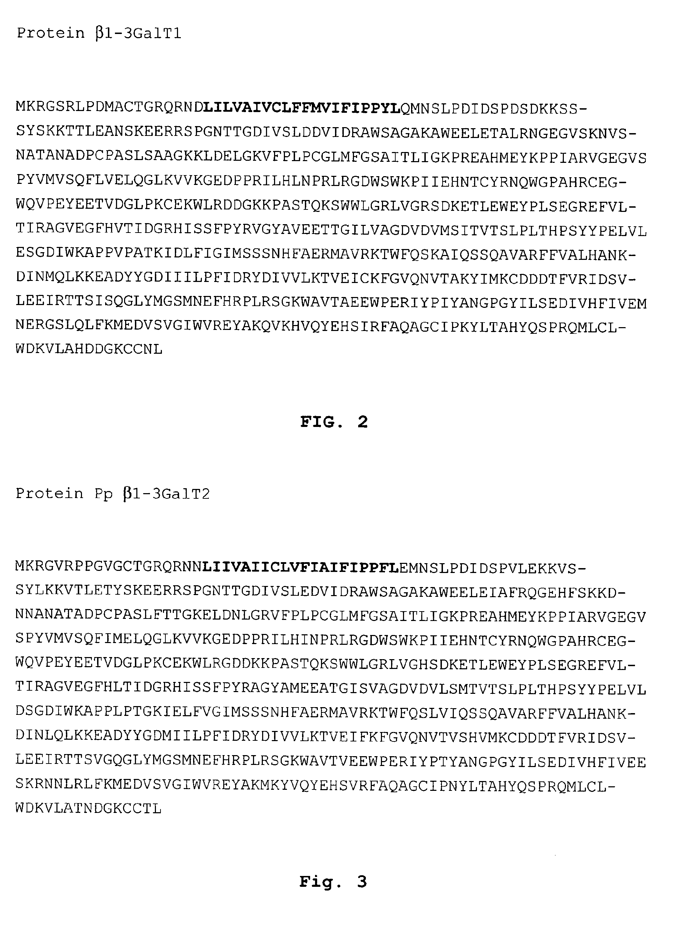

The open reading frame of the sequence having SEQ ID NO: 1 codes for a protein with 634 amino acids. The protein encoded by SEQ ID NO: 1 contains a transmembrane domain in the region between Leu20 and Leu39, and encloses the seven conserved domains - present in human β1,3-galactosyltransferases - described by Hennet (2002 Cell. Mol. Life Sci. 59, 1081-1095, Fig. 2) as well as most of the C-terminal located conserved amino acids as described by Amado et al. (1998 J. Biol. Chem. 21, 12770-12778, Fig. 2).

The open reading frame of the sequence having SEQ ID NO: 2 codes for a protein with 633 amino acids. The protein encoded by SEQ ID NO: 2 contains a transmembrane domain in the region between Leu20 and Leu39, and encloses the seven conserved domains - present in human β1,3-galactosyltransferases - described by Hennet (2002 Cell. Mol. Life Sci. 59, 1081-1095, Fig. 2) as well as most of the C-terminal located conserved amino acids as described by Amado et al. (1998 J. Biol. Chem. 21, 12770-12778, Fig. 2).

The present invention also relates to the genomic sequences of this gene as given by SEQ ID NOs. 3 or 4, of course, as all other DNA molecules or proteins according to the present invention (if not explicitly described otherwise) in isolated form.

Activity of the plant β1,3-galactosyltransferases can be analysed by different approaches.

According to Amado et al. (1998 J. Biol. Chem. 21, 12770-12778) constructs encoding the soluble secreted forms - lacking the transmembrane domain - of the β1,3-galactosyltransferases can be cloned into expression vectors e.g. appropriate for transfection of Baculo virus and amplified in Sf9 cells; the resulting expression products can be purified and subsequently assayed for β1,3-galactosyltransferase activity.

Another approach due to the analyses of specific activity can be the overexpression of the β1,3-galactosyltransferases in an appropriate host e.g. like *Physcomitrella patens* by preparing expression constructs designed to encode the full open reading frames of the β1,3-galactosyltransferases according to the present invention and by generation of *Physcomitrella* strains transgenic for at least one of the β1,3-galactosyltransferase genes according to the present invention. The generated transgenic strains show improved contents of galactosylated N-glycans. N-glycan patterns from *Physcomitrella* can be isolated and analysed as described by Koprivova et al. (2003 Plant Biol. 5, 582-591) and Koprivova et al. (2004 Plant Biotechnol. J. 2, 517-523).

β1,3-galactosyltransferase activities according to the present invention can be assayed indirectly by targeted disruption of the responsible genes in an appropriate host e.g. *Physcomitrella patens* which result in inhibition of β1,3-galactosyltransferase activities in respect to the transfer of galactose from UDP-galactose to the non-reducing terminal GlcNAc residues on N-glycans and therefore to the lack of terminal galactosylation. Again, N-glycan patterns from *Physcomitrella* can be isolated and analysed as described by Koprivova et al. (2003 Plant Biol. 5, 582-591) and Koprivova et al. (2004 Plant Biotechnol. J. 2, 517-523). Preferably, the β1,3-galactosyltransferase according to the present invention is a GlcNAc-β1,3-galactosyltransferase.

Alternatively reduction of β1,3-galactosyltransferase activity can be achieved by methods which are commonly used for this kind of purpose e.g. the well known antisense strategy, sense strategy, ribozyme technology, PNA technology or RNA interference strategy.

According to the present invention a host cell, tissue or organism is transfected with the nucleotide sequences comprising at least the sequences of SEQ ID No: 1 or SEQ ID NO: 2 which code for a functional β1,3-galactosyltransferase. In a preferred embodiment of this invention the coding sequences are linked to regulatory sequences such as promoter and termination sequences allowing expression of the β1,3-galactosyltransferase genes resulting in the expression products which show β1,3-galactosyltransferase activities. Regarding the host cell tissue or organism the regulatory sequences operably linked to the β1,3-galactosyltransferase coding sequence can be heterologous. In another embodiment the regulatory sequences operably linked to the β1,3-galactosyltransferase coding sequence can be homologous due to the used host. The regulatory sequences operably linked to the β1,3-galactosyltransferase coding sequence can be provided by the vector used for transfection or can be established *in vivo* by introducing the β1,3-galactosyltransferase coding sequence by targeted integration e.g. homologous recombination into an appropriate locus resulting in an operably functional assembly of the β1,3-galactosyltransferase coding sequence with the endogenous regulatory sequences of the host cell, tissue or organism.

In a preferred embodiment of the present invention the expression product or parts thereof e.g. a soluble form lacking transmembrane domains comprising β1,3-galactosyltransferase can be used for elongation of N-glycans on glycolipids or glycoproteins *in vitro* or *in vivo.* In a further embodiment the resulting N-glycans comprising terminal 1,3 linked galactose residues can be further elongated *in vitro* or *in vivo* with additional sugar residues like fucose, galactose or sialic acid residues. Accordingly, the present invention relates to novel glycoproteins with N-glycans sugar structure comprising complex type N-glycans containing terminal sugar residues, such as galactose, an additional fucose, sialic acid or combinations thereof. In a more preferred embodiment, these glycoproteins are surface proteins presented in the complex type N-gylcans to the outer environment of the cell,e.g. allowing protein/protein contacts (such as contacts with antibodies, other cells, etc.). Such glycoproteins produced according to the present invention are highly suitable for vaccination, especially of humans, both *in vitro* and *in vivo*.

In another embodiment of the present invention there are provided nucleotide sequences according to SEQ ID NO: 1 or SEQ ID NO: 2 which encode transmembrane domains for targeting a heterologous protein to the late Golgi cisternae. In a preferred embodiment β1,4-galactosyltransferases or sialyltransferases showing activity for elongation of N-glycans are targeted to the late Golgi cisternae by exchange of the native transmembrane domains with these of the β1,3-galactosyltransferases according to the present invention.

According to the present invention there is provided a transformed host cell that comprises at least one dysfunctional β1,3-galactosyltransferase nucleotide sequence.

In a preferred embodiment of the invention the host cell is selected from plants. In a more preferred embodiment of the present invention the host cell is selected from bryophytes including mosses and liverworts, of species from the genera Phy*scomitrella, Funaria, Sphagnum, Ceratodon, Marchantia* and *Sphaerocarpos.* The bryophyte cell is preferably from Phy*scomitrella patens.*

A preferred host according to the present invention is a bryophyte, especially *Physcomitrella patens,* a haploid non-vascular land plant, can be used for the production of glyco-engineered recombinant proteins (WO 01/25456). In *Physcomitrella* patens as well as in other plants Lewis a type structures have been detected (Koprivova et al. 2003 Plant Biol. 5, 582-591; Koprivova et al. 2004 Plant Biotechnol. J. 2, 517-523). Although from plants no β1,3-galactosyltransferases showing specific activity in elongation of N-glycan structures have been identified Phy*scomitrella* was choosen as a putative source for this unknown kind of glycosyltransferase.

The life cycle of mosses is dominated by photoautotrophic gametophytic generation. The life cycle is completely different to that of the higher plants wherein the sporophyte is the dominant generation and there are notably many differences to be observed between higher plants and bryophytes.

The gametophyte of bryophytes including mosses is characterised by two distinct developmental stages. The protonema which develops via apical growth, grows into a filamentous network of only two cell types (chloronemal and caulonemal cells). The second stage, called the gametophore, differentiates by caulinary growth from a simple apical system. Both stages are photoautotrophically active. Cultivation of protonema without differentiation into the more complex gametophore has been shown for suspension cultures in flasks as well as for bioreactor cultures (WO 01/25456). Cultivation of fully differentiated and photoautrophically active multicellullar tissue containing only a few cell types is not described for higher plants. The genetic stability of the moss cell system provides an important advantage over plant cell cultures.

There are some important differences between bryophytes (non-vascular plants) and higher plants (vascular plants) on the biochemical level. Sulfate assimilation in *Physcomitrella patens* differs significantly from that in higher plants. The key enzyme of sulfate assimilation in higher plants is adenosine 5'-phosphosulfate reductase. In *Physcomitrella patens* an alternative pathway via phosphoadenosine 5'-phosphosulfate reductase co-exists (Koprivova et al. (2002) J. Biol. Chem. 277,32195-32201). This pathway has not been characterised in higher plants.

Furthermore, many members of the bryophytes, algae and fern families produce a wide range of polyunsaturated fatty acids (Dembitsky (1993) Prog. Lipid Res. 32, 281-356). For example, arachidonic acid and eicosapentaenoic acid are thought to be produced only by lower plants and not by higher plants. Some enzymes of the metabolism of polyunsaturated fatty acids, (delta 6-acyl-group desaturase) (Girke et al. (1998), Plant J, 15, 39-48) and a component of a delta 6 elongase (Zank et al. (2002) Plant J 31, 255-268), have been cloned from *Physcomitrella patens.* No corresponding genes have been found in higher plants. This fact appears to confirm that essential differences exist between higher plants and lower plants at the biochemical level.

Moreover, bryophytes show highly efficient homologous recombination in its nuclear DNA, a unique feature for plants, which enables directed gene disruption (Girke et al. (1998) Plant J, 15, 39-48; Strepp et al. (1998) Proc Nat1 Acad Sci USA 95,4368-4373; Koprivova (2002) J. Biol. Chem.277, 32195-32201; reviewed by Reski (1999) Planta 208, 301-309; Schaefer and Zryd (2001) Plant Phys 127, 1430-1438; Schaefer (2002) Annu. Rev. Plant Biol.53, 477-501; Koprivova et al. 2004 Plant Biotechnol. J. 2, 517-523; Brucker et al. 2005 Planta 220, 864-874) further illustrating fundamental differences to higher plants. However, in some cases the use of this mechanism for altering glycosylation pattern has proven to be problematic, as shown herein in the examples. Disruption of N-acetylglucosaminyltransferase I (GNT1) in Phy*scomitrella patens* resulted in the loss of the specific transcript but only in minor differences of the N-glycosylation pattern. These results were in direct contrast to the loss of Golgi-modified complex glycans in a mutant *Arabidopsis thaliana* plant lacking GNT1 observed by von Schaewen et al. (1993) Plant Physiol 102, 1109-1118). Thus, the knockout in *Physcomitrella patens* did not result in the expected modification of the N-glycosylation pattern.

Although the knockout strategy was not successful for the glycosyltransferase GNT1, regarding the disruptions of the genes coding for the β1,2-xylosyltransferase and α1,3-galactosyltransferase knockouts were performed successfully in *Physcomitrella patens.*

In addition integration of the human β1,4-galactosyltransferase into the genome of a double knockout *Physcomitrella patens* plant resulted in a mammalian-like N-linked glycosylation pattern without the plant specific fucosyl and xylosyl residues and with mammalian-like terminal 1,4 galactosyl residues. The galactosyltransferase was found to be active.

The bryophyte cell, such as a *Physcomitrella patens* cell, can be any cell suitable for transformation according to methods of the invention as described herein, and may be a moss protoplast cell, a cell found in protonema tissue or other cell type. Indeed, the skilled addressee will appreciate that moss plant tissue comprising populations of transformed bryophyte cells according to the invention, such as transformed protonemal tissue also forms an aspect of the present invention.

"Dysfunctional" as used herein means that the nominated transferase nucleotide sequences of β1,3-galactosyltransferase (β1,3-GalT) are substantially incapable of encoding mRNA that codes for functional β1,3-GalT proteins that are capable of modifying plant N-linked glycans with 1,3 linked terminal galactose residues. In a preferment, the dysfunctional β1,3-GalT plant transferase nucleotide sequences comprise targeted insertions of exogenous nucleotide sequences into endogenous, that is genomic, native β1,3-GalT genes comprised in the nuclear bryophyte genome (whether it is a truly native bryophyte genome, that is in bryophyte cells that have not been transformed previously by man with other nucleic acid sequences, or in a transformed nuclear bryophyte genome in which nucleic acid sequence insertions have been made previously of desired nucleic acid sequences) which substantially inhibits or represses the transcription of mRNA coding for functional β1,3-GalT activity.

A further aspect of the invention relates to a biologically functional vector which comprises one of the above-indicated DNA molecules or parts thereof of differing lengths with at least 20 base pairs. For transfection into host cells, an independent vector capable of amplification is necessary, wherein, depending on the host cell, transfection mechanism, task and size of the DNA molecule, a suitable vector can be used. Since a large number of different vectors is known, an enumeration thereof would go beyond the limits of the present application and therefore is done without here, particularly since the vectors are very well known to the skilled artisan (as regards the vectors as well as all the techniques and terms used in this specification which are known to the skilled artisan, cf. also Sambrook Maniatis). Ideally, the vector has a small molecule mass and should comprise selectable genes so as to lead to an easily recognizable phenotype in a cell so thus enable an easy selection of vector-containing and vector-free host cells. To obtain a high yield of DNA and corresponding gene products, the vector should comprise a strong promoter, as well as an enhancer, gene amplification signals and regulator sequences. For an autonomous replication of the vector, furthermore, a replication origin is important. Polyadenylation sites are responsible for correct processing of the mRNA and splice signals for the RNA transcripts. If phages, viruses or virus particles are used as the vectors, packaging signals will control the packaging of the vector DNA. For instance, for transcription in plants, Ti plasmids are suitable, and for transcription in insect cells, baculoviruses, and in insects, respectively, transposons, such as the P element.

If the above-described inventive vector is inserted into a plant or into a plant cell, a post-transcriptional suppression of the gene expression of the endogenous β1,3galactosyltransferase gene is attained by transcription of a transgene homologous thereto or of parts thereof, in sense orientation. For this sense technique, furthermore, reference is made to the publications by Baucombe 1996, Plant. Mol. Biol., 9:373-382, and Brigneti et al., 1998, EMBO J. 17:6739-6746. This strategy of "gene silencing" is an effective way of suppressing the expression of the β1,3galactosyltransferase gene, cf. also Waterhouse et al., 1998, Proc. Natl. Acad. Sci. USA, 95:13959-13964.

Furthermore, the invention relates to a biologically functional vector comprising a DNA molecule according to one of the above-described embodiments, or parts thereof of differing lengths in reverse orientation to the promoter. If this vector is transfected in a host cell, an "antisense mRNA" will be read which is complementary to the mRNA of the β1,3galactosyltransferase and complexes the latter. This bond will either hinder correct processing, transportation, stability or, by preventing ribosome annealing, it will hinder translation and thus the normal gene expression of the β1,3galactosyltransferase.

Although the entire sequence of the DNA molecule could be inserted into the vector, partial sequences thereof because of their smaller size may be advantageous for certain purposes. With the antisense aspect, e.g., it is important that the DNA molecule is large enough to form a sufficiently large antisense mRNA which will bind to the transferase mRNA. A suitable antisense RNA molecule comprises, e.g., from 50 to 200 nucleotides since many of the known, naturally occurring antisense RNA molecules comprise approximately 100 nucleotides.

For a particularly effective inhibition of the expression of an active β1,3galactosyltransferase, a combination of the sense technique and the antisense technique is suitable (Waterhouse et al., 1998, Proc. Natl. Acad. Sci., USA, 95:13959-13964).

Advantageously, rapidly hybridizing RNA molecules are used. The efficiency of antisense RNA molecules which have a size of more than 50 nucleotides will depend on the annealing kinetics in vitro. Thus, e.g., rapidly annealing antisense RNA molecules exhibit a greater inhibition of protein expression than slowly hybridizing RNA molecules (Wagner et al., 1994, Annu. Rev. Microbiol., 48:713-742; Rittner et al., 1993, Nucl. Acids Res., 21:1381-1387). Such rapidly hybridizing antisense RNA molecules particularly comprise a large number of external bases (free ends and connecting sequences), a large number of structural subdomains (components) as well as a low degree of loops (Patzel et al. 1998; Nature Biotechnology, 16; 64-68). The hypothetical secondary structures of the antisense RNA molecule may, e.g., be determined by aid of a computer program, according to which a suitable antisense RNA DNA sequence is chosen.

Different sequence regions of the DNA molecule may be inserted into the vector. One possibility consists, e.g., in inserting into the vector only that part which is responsible for ribosome annealing. Blocking in this region of the mRNA will suffice to stop the entire translation. A particularly high efficiency of the antisense molecules also results for the 5'- and 3'-non-translated regions of the gene.

Preferably, the DNA molecule according to the invention includes a sequence which comprises a deletion, insertion and/or substitution mutation. The number of mutant nucleotides is variable and varies from a single one to several deleted, inserted or substituted nucleotides. It is also possible that the reading frame is shifted by the mutation. In such a "knock-out gene" it is merely important that the expression of a β1,3galactosyltransferase is disturbed, and the formation of an active, functional enzyme is prevented. In doing so, the site of the mutation is variable, as long as expression of an enzymatically active protein is prevented. Preferably, the mutation in the catalytic region of the enzyme which is located in the C-terminal region. The method of inserting mutations in DNA sequences are well known to the skilled artisan, and therefore the various possibilities of mutageneses need not be discussed here in detail. Coincidental mutageneses as well as, in particular, directed mutageneses, e.g. the site-directed mutagenesis, oligonucleotide-controlled mutagenesis or mutageneses by aid of restriction enzymes may be employed in this instance.

Alternatively, ribozyme or siRNA techniques may be applied for reducing or eliminating β1,3-GaltT activity in cells which have wildtype β1,3-GalT activity. Adaptation of siRNA techniques to the present invention are straight forward based on existing skills in the art (e.g. Nat.Reviews: RNA interference collection (October 2005)).

The invention further provides a DNA molecule which codes for a ribozyme which comprises two sequence portions of at least 10 to 15 base pairs each, which are complementary to sequence portions of an inventive DNA molecule as described above so that the ribozyme complexes and cleaves the mRNA which is transcribed from a natural β1,3galactosyltransferase DNA molecule. The ribozyme will recognized the mRNA of the β1,3galactosyltransferase by complementary base pairing with the mRNA. Subsequently, the ribozyme will cleave and destroy the RNA in a sequence-specific manner, before the enzyme is translated. After dissociation from the cleaved substrate, the ribozyme will repeatedly hybridize with RNA molecules and act as specific endonuclease. In general, ribozymes may specifically be produced for inactivation of a certain mRNA, even if not the entire DNA sequence which codes for the protein is known. Ribozymes are particularly efficient if the ribosomes move slowly along the mRNA. In that case it is easier for the ribozyme to find a ribosome-free site on the mRNA. For this reason, slow ribosome mutants are also suitable as a system for ribozymes (J. Burke, 1997, Nature Biotechnology; 15, 414-415). This DNA molecule is particularly advantageous for the downregulation and inhibition, respectively, of the expression of plant β1,3galactosyltransferases.

One possible way is also to use a varied form of a ribozmye, i.e. a minizyme. Minizymes are efficient particularly for cleaving larger mRNA molecules. A minizyme is a hammer head ribozyme which has a short oligonucleotide linker instead of the stem/loop II. Dimer-minizymes are particularly efficient (Kuwabara et al., 1998, Nature Biotechnology, 16; 961-965). Consequently, the invention also relates to a biologically functional vector which comprises one of the two last-mentioned DNA molecules (mutation or ribozyme-DNA molecule). What has been said above regarding vectors also applies in this instance. Such a vector can be, for example, inserted into a microorganism and can be used for the production of high concentrations of the above described DNA molecules. Furthermore such a vector is particularly good for the insertion of a specific DNA molecule into a plant organism in order to downregulate or completely inhibit the β1,3galactosyltransferase production in this organism. All vectors described above can also be made with genomic sequences of β1,3-GalT genes, such as SEQ ID NOs. 3 or 4.

Bryophyte cells of the invention or ancestors thereof may be any which have been transformed previously with heterologous genes of interest that code for primary sequences of proteins of interest which are glycosylated with mammalian glycosylation patterns as described herein. Preferably, the glycosylation patterns are of the human type. Alternatively, the bryophyte cell may be transformed severally, that is, simultaneously or over time with nucleotide sequences coding for at least a primary protein sequence of interest, typically at least a pharmaceutical protein of interest for use in humans or mammals such as livestock species including bovine, ovine, equine and porcine species, that require mammalian glycosylation patterns to be placed on them in accordance with the methods of the invention as described herein. Such pharmaceutical glycoproteins for use in mammals, including man include but are not limited to proteins such as VEGF, interferons such as α-interferon, β-interferon, gamma-interferon, blood-clotting factors selected from Factor VII, VIII, IX, X, XI, and XII, fertility hormones including luteinising hormone, follicle stimulating hormone growth factors including epidermal growth factor, platelet-derived growth factor, granulocyte colony stimulating factor and the like, prolactin, oxytocin, thyroid stimulating hormone, adrenocorticotropic hormone, calcitonin, parathyroid hormone, somatostatin, erythropoietin (EPO), enzymes such as β-glucocerebrosidase, haemoglobin, collagen, fusion proteins such as the fusion protein of TNF αreceptor ligand binding domain with Fc portion of IgG and the like. Furthermore, the method of the invention can be used for the production of immunglobulins such as antibodies such as specific monoclonal antibodies or active fragments thereof.

Detailed information on the culturing of mosses which are suitable for use in the invention, such as *Leptobryum pyriforme* and *Sphagnum magellanicum* in bioreactors, is known in the prior art (see, for example, E. Wilbert, "Biotechnological studies concerning the mass culture of mosses with particular consideration of the arachidonic acid metabolism", Ph.D. thesis, University of Mainz (1991); H. Rudolph and S. Rasmussen, Studies on secondary metabolism of Sphagnum cultivated in bioreactors, Crypt. Bot., 3, pp. 67-73 (1992)). Especially preferred for the purposes of the present invention is the use of *Physcomitrella patens,* since molecular biology techniques are practised on this organism (for a review see R. Reski, Development, genetics and molecular biology of mosses, Bot. Acta, 111, pp. 1-15 (1998)).

Suitable transformation systems have been developed for the biotechnological exploitation of *Physcomitrella* for the production of heterologous proteins. For example, successful transformations have been carried out by direct DNA transfer into protonema tissue using particle guns. PEG-mediated DNA transfer into moss protoplasts has also been successfully achieved. The PEG-mediated transformation method has been described many times for *Physcomitrella patens* and leads both to transient and to stable transformants (see, for example, K. Reutter and R. Reski, Production of a heterologous protein in bioreactor cultures of fully differentiated moss plants, Pl. Tissue culture and Biotech., 2, pp. 142-147 (1996)).

In a further embodiment of the present invention there is provided a method of producing at least a bryophyte cell wherein β-1,3-GalT activity is substantially reduced that comprises introducing into the said cell i) a first nucleic acid sequence that is specifically targeted to the endogenous β1,3 encoding nucleotide sequence according to SEQ ID NO: 1 and ii) a second nucleic acid sequence that is specifically targeted to the endogenous β1,3 encoding nucleotide sequence according to SEQ ID NO: 2.

The skilled addressee will appreciate that the order of introduction of said first and second transferase nucleic acid sequences into the bryophyte cell is not important: it can be performed in any order. The first and second nucleic acid sequences can be targeted to specific portions of the endogenous, native β1,3-GalT genes located in the nuclear genome of the bryophyte cell defined by specific restriction enzyme sites thereof, for example, according to the examples as provided herein. By specifically targeting the sequences of the native β1,3-GalT genes with nucleotide sequences that specifically integrate with the target native transferase genes of interest, the expression of the said sequences is substantially impaired if not completely disrupted.

Preferably all glycosylated mammalian proteins mentioned hereinabove are of the human type. Other proteins that are contemplated for production in the present invention include proteins for use in veterinary care and may correspond to animal homologues of the human proteins mentioned herein.

An exogenous promoter is one that denotes a promoter that is introduced in front of a nucleic acid sequence of interest and is operably associated therewith. Thus an exogenous promoter is one that has been placed in front of a selected nucleic acid component as herein defined and does not consist of the natural or native promoter usually associated with the nucleic acid component of interest as found in wild type circumstances. Thus a promoter may be native to a bryophyte cell of interest but may not be operably associated with the nucleic acid of interest in front in wild-type bryophyte cells. Typically, an exogenous promoter is one that is transferred to a host bryophyte cell from a source other than the host cell.

Regarding the production of N-glycan structures with improved β1,3-galactosylation the cDNA's encoding the β-1,3-GalT proteins, the glycosylated and the mammalian proteins as described herein contain at least one type of promoter that is operable in a bryophyte cell, for example, an inducible or a constitutive promoter operatively linked to a β-1,3-GalT nucleic acid sequence and/or second nucleic acid sequence for a glycosylated mammalian protein as herein defined and as provided by the present invention. As discussed, this enables control of expression of the gene(s).

The term "inducible" as applied to a promoter is well understood by those skilled in the art. In essence, expression under the control of an inducible promoter is "switched on" or increased in response to an applied stimulus (which may be generated within a cell or provided exogenously). The nature of the stimulus varies between promoters. Some inducible promoters cause little or undetectable levels of expression (or no expression) in the absence of the appropriate stimulus. Other inducible promoters cause detectable constitutive expression in the absence of the stimulus. Whatever the level of expression is in the absence of the stimulus, expression from any inducible promoter is increased in the presence of the correct stimulus. The preferable situation is where the level of expression increases upon application of the relevant stimulus by an amount effective to alter a phenotypic characteristic. Thus an inducible (or "switchable") promoter may be used which causes a basic level of expression in the absence of the stimulus which level is too low to bring about a desired phenotype (and may in fact be zero). Upon application of the stimulus, expression is increased (or switched on) to a level, which brings about the desired phenotype.

As alluded to herein, bryophyte expression systems are also known to the man skilled in the art. A bryophyte promoter, in particular a *Physcomitrella patens* promoter, is any DNA sequence capable of binding a host DNA-dependent RNA polymerase and initiating the downstream (3') transcription of a coding sequence (e.g. structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A bryophyte promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

The skilled addressee will appreciate that bryophyte promoter sequences encoding enzymes in bryophyte metabolic pathways can provide particularly useful promoter sequences.

In addition, synthetic promoters which do not occur in nature may also function as bryophyte promoters. For example, UAS sequences of one byrophyte promoter may be joined with the transcription activation region of another bryophyte promoter, creating a synthetic hybrid promoter. An example of a suitable promoter is the one used in the TOP 10 expression system for *Physcomitrella patens* by Zeidler et al. (1996) Plant. Mol. Biol. 30, 199-205). Furthermore, a bryophyte promoter can include naturally occurring promoters of non-bryophyte origin that have the ability to bind a bryophyte DNA-dependent RNA polymerase and initiate transcription. Examples of such promoters include those described, inter alia, the rice P-Actin 1 promoter and the Chlamydomonas RbcS promoter (Zeidler et al. (1999) J. Plant Physiol. 154, 641-650), Cohen et al., Proc. Natl. Acad. Sci. USA, 77: 1078, 1980; Henikoff et al., Nature, 283: 835, 1981; Hollenberg et al., Curr. Topics Microbiol. Immunol., 96: 119, 1981; Hollenberg et al., "The Expression of Bacterial Antibiotic Resistance Genes in the Yeast Saccharomyces cerevisiae", in: Plasmids of Medical, Environmental and Commercial Importance (eds. K. N. Timms and A. Puhler), 1979; Mercerau-Puigalon et al., Gene, 1 1: 163, 1980; Panthier et al., Curr. Genet., 2: 109, 1980.

The DNA molecules according to the present invention may be expressed intracellularly in bryophytes. A promoter sequence may be directly linked with the DNA molecule, in which case the first amino acid at the N-terminus of the recombinant protein will always be a methionine, which is encoded by the AUG start codon on the mRNA. If desired, methionine at the N-terminus may be cleaved from the protein by in vitro incubation with cyanogen bromide.

Alternatively, foreign proteins can also be secreted from the bryophyte cell into the growth media by creating chimeric DNA molecules that encode a fusion protein comprised of a leader sequence fragment that provides for secretion in or out of bryophyte cells of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted bryophyte proteins, such as leaders of non-bryophyte origin, such as a VEGF leader, exist that may also provide for secretion in bryophyte cells.

Transcription termination sequences that are recognized by and functional in bryophyte cells are regulatory regions located 3' to the translation stop codon, and thus together with the promoter flank the coding sequence. These sequences direct the transcription of an mRNA which can be translated into the polypeptide encoded by the DNA. An example of a suitable termination sequence that works in *Physcomitrella patens* is the termination region of Cauliflower mosaic virus.

Typically, the components, comprising a promoter, leader (if desired), coding sequence of interest, and transcription termination sequence, are put together into expression constructs of the invention. Expression constructs are often maintained in a DNA plasmid, which is an extrachromosomal element capable of stable maintenance in a host, such as a bacterium. The DNA plasmid may have two origins of replication, thus allowing it to be maintained, for example, in a bryophyte for expression and in a prokaryotic host for cloning and amplification. Generally speaking it is sufficient if the plasmid has one origin of replication for cloning and amplification in a prokaryotic host cell. In addition, a DNA plasmid may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Either a high or low copy number vector may be selected, depending upon the effect of the vector and the foreign protein on the host (see, e.g., Brake et al., supra).

Alternatively, the expression constructs can be integrated into the bryophyte genome with an integrating vector. Integrating vectors usually contain at least one sequence homologous to a bryophyte chromosome that allows the vector to integrate, and preferably contain two homologous sequences flanking the expression construct. An integrating vector may be directed to a specific locus in moss by selecting the appropriate homologous sequence for inclusion in the vector as described and exemplified herein. One or more expression constructs may integrate. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of bryophyte cells that have been transformed.

Selectable markers may include biosynthetic genes that can be expressed in the moss host, such as the G418 or hygromycin B resistance genes, which confer resistance in bryophyte cells to G418 and hygromycin B, respectively. In addition, a suitable selectable marker may also provide bryophyte cells with the ability to grow in the presence of toxic compounds, such as metal.

Alternatively, some of the above-described components can be put together into transformation vectors. Transformation vectors are usually comprised of a selectable marker that is either maintained in a DNA plasmid or developed into an integrating vector, as described above.

Alternatively, by achieving high yields of transformation events as observed in *Physcomitrella* the use of markers for the selection of transformation events can be avoided.

Methods of introducing exogenous DNA into bryophyte cells are well-known in the art, and are described inter alia by Schaefer D. G. "Principles and protocols for the moss Physcomitrella patens", (May 2001) Institute of Ecology, Laboratory of Plant Cell Genetics, University of Lausanne; Reutter K. and Reski R., Plant Tissue Culture and Biotechnology September 1996, Vol.2, No.3; Zeidler M et al., (1996), Plant Molecular Biology 30:199-205.

Those skilled in the art are well able to construct vectors and design protocols for recombinant nucleic acid sequence or gene expression as described above. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: a Laboratory Manual: 2nd edition, Sambrook et al, 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1992. The disclosures of Sambrook et al. and Ausubel et al. are incorporated herein by reference.

As described above, selectable genetic markers may facilitate the selection of transgenic bryophyte cells and these may consist of chimaeric genes that confer selectable phenotypes as alluded to herein.

When introducing selected glycosyltransferase encoding nucleic acid sequences and polypetide sequences comprising glycosyltransferase activity into a bryophyte cell, certain considerations must be taken into account, well known to those skilled in the art. The nucleic acid(s) to be inserted should be assembled within a construct, which contains effective regulatory elements, which will drive transcription. There must be available a method of transporting the construct into the cell. Once the construct is within the cell membrane, integration into the endogenous chromosomal material either will or will not occur.

The invention further encompasses a host cell transformed with vectors or constructs as set forth above, especially a bryophyte or a microbial cell. Thus, a host cell, such as a bryophyte cell, including nucleotide sequences of the invention as herein indicated is provided. Within the cell, the nucleotide sequence may be incorporated within the chromosome.

Also according to the invention there is provided a bryophyte cell having incorporated into its genome at least a nucleotide sequence, particularly heterologous nucleotide sequences, as provided by the present invention under operative control of regulatory sequences for control of expression as herein described. The coding sequence may be operably linked to one or more regulatory sequences which may be heterologous or foreign to the nucleic acid sequences employed in the invention, such as not naturally associated with the nucleic acid sequence(s) for its(their) expression. The nucleotide sequence according to the invention may be placed under the control of an externally inducible promoter to place expression under the control of the user. A further aspect of the present invention provides a method of making such a bryophyte cell, particularly a Phy*scomitrella* patens cell involving introduction of nucleic acid sequence(s) contemplated for use in the invention or at least a suitable vector including the sequence(s) contemplated for use in the invention into a bryophyte cell and causing or allowing recombination between the vector and the bryophyte cell genome to introduce the said sequences into the genome. The invention extends to bryophyte cells, particularly *Physcomitrella patens* cells containing a GalT nucleotide and/or a nucleotide sequence coding for a polypeptide sequence destined for the addition of a mammalian glycosylation pattern thereto and suitable for use in the invention as a result of introduction of the nucleotide sequence into an ancestor cell.

The term "heterologous" may be used to indicate that the gene/sequence of nucleotides in question have been introduced into bryophyte cells or an ancestor thereof, using genetic engineering, ie by human intervention. A transgenic bryophyte cell, i.e. transgenic for the nucleotide sequence in question, may be provided. The transgene may be on an extra-genomic vector or incorporated, preferably stably, into the genome. A heterologous gene may replace an endogenous equivalent gene, ie one that normally performs the same or a similar function, or the inserted sequence may be additional to the endogenous gene or other sequence. An advantage of introduction of a heterologous gene is the ability to place expression of a sequence under the control of a promoter of choice, in order to be able to influence expression according to preference. Nucleotide sequences heterologous, or exogenous or foreign, to a bryophyte cell may be non-naturally occurring in cells of that type, strain or species. Thus, a nucleotide sequence may include a coding sequence of or derived from a particular type of bryophyte cell, such as a *Physcomitella patens* cell, placed within the context of a bryophyte cell of a different type or species. A further possibility is for a nucleotide sequence to be placed within a bryophyte cell in which it or a homologue is found naturally, but wherein the nucleotide sequence is linked and/or adjacent to nucleic acid which does not occur naturally within the cell, or cells of that type or species or strain, such as operably linked to one or more regulatory sequences, such as a promoter sequence, for control of expression. A sequence within a bryophyte or other host cell may be identifiably heterologous, exogenous or foreign.

The present invention also encompasses the desired polypeptide expression product of the combination of nucleic acid molecules according to the invention as disclosed herein or obtainable in accordance with the information and suggestions herein. Also provided are methods of making such an expression product by expression from nucleotide sequences encoding therefore under suitable conditions in suitable host cells e.g. E.coli. Those skilled in the art are well able to construct vectors and design protocols and systems for expression and recovery of products of recombinant gene expression.

A polypeptide according to the present invention may be an allele, variant, fragment, derivative, mutant or homologue of the(a) polypeptides as mentioned herein. The allele, variant, fragment, derivative, mutant or homologue may have substantially the same function of the polypeptides alluded to above and as shown herein or may be a functional mutant thereof. In the context of pharmaceutical proteins as described herein for use in humans, the skilled addressee will appreciate that the primary sequence of such proteins and their glycosylation pattern will mimick or preferably be identical to that found in humans.

"Identity" in relation to a nucleic acid sequence or to an amino acid sequence of the invention may be used to refer to identity of the whole sequence or essential parts thereof. As noted already above, high level of amino acid identity may be limited to functionally significant domains or regions, e.g. any of the domains identified herein.

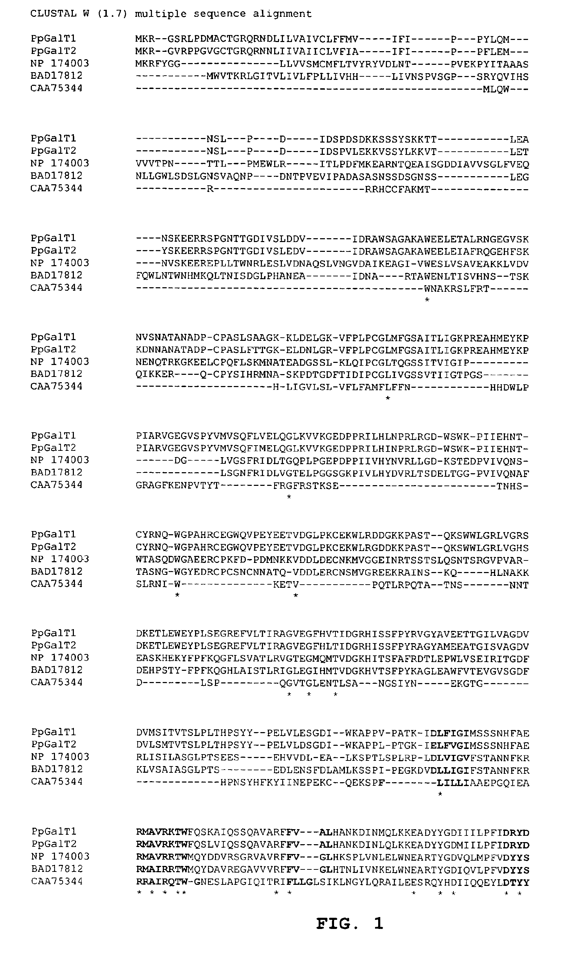

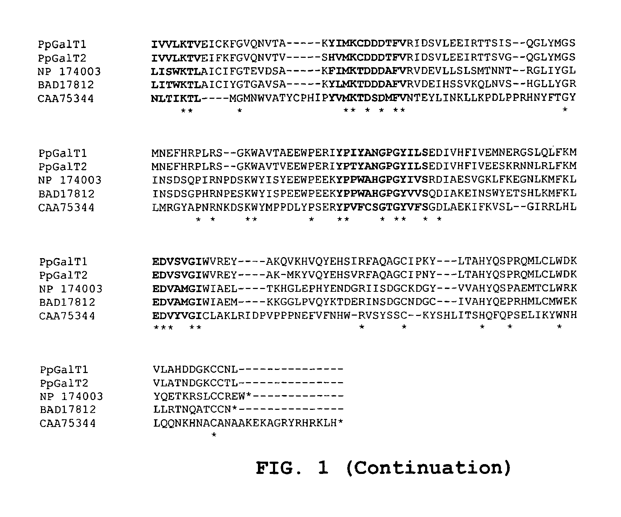

In particular, homologues of the particular bryophyte-derived polypeptide sequences provided herein, are provided by the present invention, as are mutants, variants, fragments and derivatives of such homologues. Thus the present invention also extends to polypeptides which include amino acid sequences with β1,3-galactosyltransferases function as defined herein and as obtainable using sequence information as provided herein. The β1,3-galactosyltransferase according to the present invention may at the amino acid level have identity with the amino acid sequences of the sequences disclosed herein, especially of PpGalT1 or PpGalT2, of at least about 50%, or at least 55%, or at least about 60%, or at least about 65%, or at least about 70%, or at least about 75%, or at least about 80% identity, or at least about 85 %, or at least about 88% identity, or at least about 90% identity and most preferably at least about 95% or greater identity provided that such proteins have a β1,3-galactosyltransferase activity that fits within the context of the present invention. The %identity mentioned should be preferably given in the region comprising the seven conserved domains as depicted in Fig. 1 (including appropriate "-" as being obvious occurring to the skilled man in the art) when comparing the sequences in question to e.g. either PpGalT1 or PpGalT2).

In certain embodiments, an allele, variant, derivative, mutant derivative, mutant or homologue of the specific sequence may show little overall identity, say about 20%, or about 25%, or about 30%, or about 35%, or about 40% or about 45%, with the specific sequence. However, in functionally significant domains or regions, the amino acid identity may be much higher. Putative functionally significant domains or regions can be identified using processes of bioinformatics, including comparison of the sequences of homologues. Preferred β1,3-GalT proteins according to the present invention show more than 80%, especially more than 90% identity in the seven conserved domains according to Fig. 1 (amino acid residues in bold), especially preferred with the conserved amino acids (represented by a "*" (star) in Fig. 1) being completely (or at least to a 95% extent) present. Specifically preferred variants of the β1,3-GalT according to the present invention comprise more than 80%, preferably more than 90%, especially 100%, of the conserved amino acids as depicted in Fig. 1.

Functionally significant domains or regions of different polypeptides may be combined for expression from encoding nucleic acid as a fusion protein. For example, particularly advantageous or desirable properties of different homologues may be combined in a hybrid protein, such that the resultant expression product, with β1,3-galactosyltransferase function, may include fragments of various parent proteins, if appropriate.

Identity may easily be calculated as % value of aligned sequences (including intelligent "-"). Similarity of amino acid sequences may be as defined and determined by the TBLASTN program, of Altschul et al. (1990) J. Mol. Biol. 215: 403-10, which is in standard use in the art. In particular, TBLASTN 2.0 may be used with Matrix BLOSUM62 and GAP penalties: existence: 11, extension: 1. Another standard program that may be used is BestFit, which is part of the Wisconsin Package, Version 8, September 1994, (Genetics Computer Group, 575 Science Drive, Madison, Wisconsin, USA, Wisconsin 53711). BestFit makes an optimal alignment of the best segment of similarity between two sequences. Optimal alignments are found by inserting gaps to maximize the number of matches using the local identity algorithm of Smith and Waterman (Adv. Appl. Math. (1981) 2: 482-489). Other algorithms include GAP, which uses the Needleman and Wunsch algorithm to align two complete sequences that maximizes the number of matches and minimizes the number of gaps. As with any algorithm, generally the default parameters are used, which for GAP are a gap creation penalty = 12 and gap extension penalty = 4. Alternatively, a gap creation penalty of 3 and gap extension penalty of 0.1 may be used. The algorithm FASTA (which uses the method of Pearson and Lipman (1988) PNAS USA 85: 2444-2448) is a further alternative.

An advantageous method of producing recombinant host cells, in particular plant cells, or plants, respectively, consists in that the DNA molecule according to the present invention, especially comprising an inactivating mutation is inserted into the genome of the host cell, or plant, respectively, in the place of the non-mutant homologous sequence (Schaefer et al., 1997, Plant J.; 11(6):1195-1206). This method thus does not function with a vector, but with a pure DNA molecule. The DNA molecule according to the present invention is inserted into the host e.g. by gene bombardment, microinjection or PEG-mediated direct DNA transfer, to mention just three examples. This DNA molecule binds to the homologous sequence in the genome of the host so that a homologous recombination and thus reception of the deletion, insertion or substitution mutation, respectively, will result in the genome: Expression of the β1,3-galactosyltransferase can e.g. be suppressed or completely blocked, respectively.

A further aspect of the invention relates to plants, plant tissues or plant cells, respectively their β1,3galactosyltransferase activity being less than 50%, in particular less than 20%, particularly preferred 0%, of the β1,3galactosyltransferase activity occurring in natural plants or plant cells, The advantage of these plants or plant cells, respectively, is that the glycoproteins produced by them do not comprise any or hardly comprise any β1,3-bound galactose. If products of these plants, respectively, are taken up by human or vertebrate bodies, there will be no immune reaction to the β1,3 linked galactose epitope.
Preferably, recombinant plants or plant cells, respectively, are provided which have been prepared by one of the methods described above, their β1,3-galactosyltransferase production being suppressed or completely blocked, respectively.

The invention also relates to a PNA molecule comprising a base sequence complementary to the sequence of the DNA molecule according to the invention as well as partial sequences thereof. PNA (peptide nucleic acid) is a DNA-like sequence, the nucleobases being bound to a pseudo-peptide backbone. PNA generally hybridizes with complementary DNA-, RNA- or PNA-oligomers by Watson-Crick base pairing and helix formation. The peptide backbone ensures a greater resistance to enzymatic degradation. The PNA molecule thus is an improved antisense agent. Neither nucleases nor proteases are capable of attacking a PNA molecule. The stability of the PNA molecule, if bound to a complementary sequence, comprises a sufficient steric blocking of DNA and RNA polymerases, reverse transcriptase, telomerase and ribosomes. If the PNA molecule comprises the above-mentioned sequence, it will bind to the DNA or to a site of the DNA, respectively, which codes for β1,3galactosyltransferase and in this way is capable of inhibiting transcription of this enzyme. As it is neither transcribed nor translated, the PNA molecule will be prepared synthetically, e.g. by aid of the the t-Boc technique. Advantageously, a PNA molecule is provided which comprises a base sequence which corresponds to the sequence of the inventive DNA molecule as well as partial sequences thereof. This PNA molecule will complex the mRNA or a site of the mRNA of β1,3-galactosyltransferase so that the translation of the enzyme will be inhibited. Similar arguments as set forth for the antisense RNA apply in this case. Thus, e.g., a particularly efficient complexing region is the translation start region or also the 5'-non-translated regions of mRNA.

A further aspect of the present invention relates to a method of preparing plants, tissues, or cells, respectively, in particular plant cells which comprise a blocked expression of the β1,3galactosyltransferase on transcription or translation level, respectively, which is characterized in that inventive PNA molecules are inserted in the cells. To insert the PNA molecule or the PNA molecules, respectively, in the cell, again conventional methods, such as, e.g., electroporation or microinjection, are used. Particularly efficient is insertion if the PNA oligomers are bound to cell penetration peptides, e.g. transportan or pAntp (Pooga et al., 1998, Nature Biotechnology, 16; 857-861).

The invention provides a method of preparing recombinant glycoproteins which is characterized in that the inventive, recombinant plants or plant cells, respectively, whose β1,3-galactosyltransferase production is suppressed or completely blocked, respectively, or plants, or tissues, or cells, respectively, in which the PNA molecules have been inserted according to the method of the invention, are transfected with the gene that expresses the glycoprotein so that the recombinant glycoproteins are expressed. In doing so, as has already been described above, vectors comprising genes for the desired proteins are transfected into the host or host cells, respectively, as has also already been described above. The transfected plant cells will express the desired proteins, and they have no or hardly any β1,3-bound galactose. Thus, they do not trigger the immune reactions already mentioned above in the human or vertebrate body. Any proteins may be produced in these systems.
Advantageously, a method of preparing recombinant human glycoproteins is provided which is characterized in that the recombinant plants or plant cells, respectively, whose β1,3-galactosyltransferase production is suppressed or completely blocked, or plants, or tissues, or cells, respectively, in which PNA molecules have been inserted according to the method of the invention, are transfected with the gene that expresses the glycoprotein so that the recombinant glycoproteins are expressed. By this method it becomes possible to produce human proteins in plants (plant cells) which, if taken up by the human body, do not trigger any immune reaction directed against β1,3-bound galacatase residues. There, it is possible to utilize plant types for producing the recombinant glycoproteins which serve as food stuffs, e.g. banana, potato and/or tomato. The tissues of this plant comprise the recombinant glycoprotein so that, e.g. by extraction of the recombinant glycoprotein from the tissue and subsequent administration, or directly by eating the plant tissue, respectively, the recombinant glycoprotein is taken up in the human body. Preferably, a method of preparing recombinant human glycoproteins for medical use is provided, wherein the inventive, recombinant plants or plant cells, respectively, whose β1,3-galactosyltransferase production is suppressed or completely blocked, respectively, or plants, or tissues, or cells, respectively, into which the PNA molecules have been inserted according to the method of the invention, are transfected with the gene that expresses the glycoprotein so that the recombinant glycoproteins are expressed. In doing so, any protein can be used which is of medical interest.

Moreover, the present invention relates to recombinant glycoproteins according to a method described above, wherein they have been prepared in plant systems and wherein their peptide sequence comprises less than 50%, in particular less than 20%, particularly preferred 0%, of the β1,3-bound galactose residues occurring in proteins expressed in non-galactosyltransferase-reduced plant systems. Naturally, glycoproteins which do not comprise β1,3-bound galactose residues are to be preferred. The amount of β1,3-bound galactose will depend on the degree of the above-described suppression of the β1,3-galactosyltransferase.

Preferably, the invention relates to recombinant human glycoproteins which have been produced in plant systems according to a method described above and whose peptide sequence comprises less than 50%, in particular less than 20%, particularly preferred 0%, of the β1,3-bound galactose residues occurring in the proteins expressed in non-galactosyltransferase-reduced plant or systems.

A particularly preferred embodiment relates to recombinant human glycoproteins for medical use which have been prepared in plant systems according to a method described above and whose peptide sequence comprises less than 50%, in particular less than 20%, particularly preferred 0%, of the β1,3-bound galactose residues occurring in the proteins expressed in non-galactosyltransferase-reduced plant systems.

A further aspect comprises a pharmaceutical composition comprising the glycoproteins according to the invention. In addition to the glycoproteins of the invention, the pharmaceutical composition comprises further additions common for such compositions. These are, e.g., suitable diluting agents of various buffer contents (e.g. Tris-HCl, acetate, phosphate, pH and ionic strength, additives, such as tensides and solubilizers (e.g. Tween 80, Polysorbate 80), preservatives (e.g. Thimerosal, benzyl alcohol), adjuvants, antioxidants (e.g. ascorbic acid, sodium metabisulfite), emulsifiers, fillers (e.g. lactose, mannitol), covalent bonds of polymers, such as polyethylene glycol, to the protein, incorporation of the material in particulate compositions of polymeric compounds, such as polylactic acid, polyglycolic acid, etc. or in liposomes, auxiliary agents and/or carrier substances which are suitable in the respective treatment. Such compositions will influence the physical condition, stability, rate of in vivo liberation and rate of in vivo excretion of the glycoproteins of the invention.

The invention also provides a method of selecting DNA molecules which code for a β1,3-galactosyltransferase, in a sample, wherein the labelled DNA molecules of the invention are admixed to the sample, which bind to the DNA molecules that code for a β1,3-galactosyltransferase. The hybridized DNA molecules can be detected, quantitated and selected. For the sample to contain single strand DNA with which the labelled DNA molecules can hybridize, the sample is denatured, e.g. by heating.

One possible way is to separate the DNA to be assayed, possibly after the addition of endonucleases, by gel electrophoresis on an agarose gel. After having been transferred to a membrane of nitrocellulose, the labelled DNA molecules according to the invention are admixed which hybridize to the corresponding homologous DNA molecule ("Southern blotting").

Another possible way consists in finding homologous genes from other species by PCR-dependent methods using specific and/or degenerated primers, derived from the sequence of the DNA molecule according to the invention.

Preferably, the sample for the above-identified inventive method comprises genomic DNA of a plant organism. By this method, a large number of plants is assayed in a very rapid and efficient manner for the presence of the β1,3-galactosyltransferase gene. In this manner, it is respectively possible to select plants which do not comprise this gene, or to suppress or completely block, respectively, the expression of the β1,3-galactosyltransferase in such plants which comprise this gene, by an above-described method of the invention, so that subsequently they may be used for the transfection and production of (human) glycoproteins.

The invention also relates to DNA molecules which code for a β1,3-galactosyltransferase which have been selected according to the two last-mentioned methods and subsequently have been isolated from the sample. These molecules can be used for further assays. They can be sequenced and in turn can be used as DNA probes for finding β1,3-galactosyltransferases. These - labelled - DNA molecules will function for organisms, which are related to the organisms from which they have been isolated, more efficiently as probes than the DNA molecules of the invention.

The invention also relates to a method of preparing "plantified" carbohydrate units of human and other vertebrate glycoproteins, wherein fucose units as well as β1,3galactosyltransferase encoded by an above-described DNA molecule are admixed to a sample that comprises a carbohydrate unit or a glycoprotein, respectively, so that galactase in β1,3-position will be bound by the β1,3galactosyltransferase to the carbohydrate unit or to the glycoprotein, respectively. By the method according to the invention for cloning β1,3galactosyltransferase it is possible to produce large amounts of purified enzyme. To obtain a fully active transferase, suitable reaction conditions are provided.

The invention will be explained in more detail by way of the following examples and drawing figures to which, of course, it shall not be restricted.

Fig. 1 shows an amino acid alignment of β1,3-GalT. The seven conserved domains of β1,3-galactosyltransferases are indicated in bold letters. Conserved amino acid residues are indicated by stars. Similarities according to the reference sequence from humans (CAA75344, β1,3-galactosyltransferase from humans) are predicted as follows BAD17812 (putative β1,3-galactosyltransferase from Oryza sativa) = 17%; NP 174003 (putative β1,3-galactosyltransferase from Arabidopsis thaliana) = 16%; PpGalT1 (β1,3-galactosyltransferase 1 from Physcomitrella patens) = 15%; PpGalT2 (β1,3-galactosyltransferase 2 from Physcomitrella patens) = 16%;

Fig. 2 shows the protein sequence predicted from the coding DNA sequence of the β1,3-galactosyltransferase 1 gene from Physcomitrella patens. The transmembrane domain is indicated in bold letters; and

Fig 3 shows the protein sequence predicted from the coding DNA sequence of the β1,3-galactosyltransferase 2 gene from Physcomitrella patens. The transmembrane domain is indicated in bold letters.

### Examples

### Methods and Materials

### Plant material

A glyco-engineered double knockout strain of *Physcomitrella patens* lacking fucose and xylose residues in the core structure of N-glycans was used (Koprivova et al. 2004 Plant Biotechnol. J. 2, 517-523).

### Standard culture conditions

Plants were grown axenicallly under sterile conditions in plain inorganic liquid modified Knop medium (1000 mg/l Ca(NO₃)₂ x 4H₂O 250 mg/l KCl, 250 mg/l KH₂PO4, 250 mg/l MgSO₄ x 7 H₂O and 12.5 mg/l FeSO₄ X 7 H₂O; pH 5.8 (Reski and Abel (1985) Planta 165, 354-358). Plants were grown in 500 ml Erlenmeyer flasks containing 200 ml of culture medium and flasks were shaken on a Certomat R shaker (B.Braun Biotech International, Germany) set at 120 rpm. Conditions in the growth chamber were 25 +/- 3°C and a light-dark regime of 16:8 h. The flasks were illuminated from above by two fluorescent tubes (Osram L 58 W / 25) providing 35 micromols⁻¹m⁻². The cultures were subcultured once a week by disintegration using an Ultra-Turrax homogenizer (IKA, Staufen, Germany) and inoculation of two new 500 ml Erlenmeyer flasks containing 100 ml fresh Knop medium.

### Protoplast isolation

After filtration the moss protonemata were preincubated in 0.5 M mannitol. After 30 min, 4 % Driselase (Sigma, Deisenhofen, Germany) was added to the suspension. Driselase was dissolved in 0.5 M mannitol (pH 5.6-5.8), centrifuged at 3600 rpm for 10 min and sterilised by passage through a 0.22 microm filter (Millex GP, Millipore Corporation, USA). The suspension, containing 1% Driselase (final concentration), was incubated in the dark at RT and agitated gently (best yields of protoplasts were achieved after 2 hours of incubation) (Schaefer, "Principles and protocols for the moss Physcomitrella patens", (May 2001) Institute of Ecology, Laboratory of Plant Cell Genetics, University of Lausanne. The suspension was passed through sieves (Wilson, CLF, Germany) with pore sizes of 100 microm and 50 microm. The suspension was centrifuged in sterile centrifuge tubes and protoplasts were sedimented at RT for 10 min at 55 g (acceleration of 3; slow down at 3; Multifuge 3 S-R, Kendro, Germany) (Schaefer, supra). Protoplasts were gently resuspended in 3M medium(15 mM MgCl₂ x 2H₂O; 0.1 % MES; 0.48 M mannitol; pH 5.6; 540 mOsm; sterile filtered, Schaefer et al. (1991) Mol Gen Genet 226, 418-424). The suspension was centrifuged again at RT for 10 min at 55 g (acceleration of 3; slow down at 3; Multifuge 3 S-R, Kendro, Germany). Protoplasts were gently resuspended in 3M medium (15 mM MgCl₂ x 2H₂O; 0.1 % MES; 0.48 M mannitol; pH 5.6; 540 mOsm; sterile filtered, Schaefer et al. (1991) Mol Gen Genet 226, 418-424). For counting protoplasts a small volume of the suspension was transferred to a Fuchs-Rosenthal-chamber.

### Transformation protocol

For transformation protoplasts were incubated on ice in the dark for 30 minutes. Subsequently, protoplasts were sedimented by centrifugation at RT for 10 min at 55 g (acceleration of 3; slow down at 3; Multifuge 3 S-R, Kendro). Protoplasts were resuspended in 3M medium (15 mM MgCl₂ x 2H₂O; 0.1 % MES; 0.48 M mannitol; pH 5.6; 540 mOsm; sterile filtered, Schaefer et al. (1991) Mol Gen Genet 226, 418-424) at a concentration of 1.2 x 10⁶ protoplasts / ml (Reutter and Reski (1996) Production of a heterologous protein in bioreactor cultures of fully differentiated moss plants, Pl. Tissue culture and Biotech., 2, pp. 142-147). 25 microlitre of this protoplast suspension were dispensed into a new sterile centrifuge tube, 5 microlitre DNA solution (column purified DNA in H₂O (Qiagen, Hilden, Germany); 10-100 microlitre; optimal DNA amount of 6 microgram) was added and finally 25 microlitre PEG-solution (40% PEG 4000; 0.4 M mannitol; 0.1 M Ca(NO₃)₂; pH 6 after autoclaving) was added. The suspension was immediately but gently mixed and then incubated for 6 min at RT with occasional gentle mixing. The suspension was diluted progressively by adding 1, 2, 3 and 4 ml of 3M medium. The suspension was centrifuged at 20°C for 10 minutes at 55 g (acceleration of 3; slow down at 3; Multifuge 3 S-R, Kendro). The pellet was resuspended in 3 ml regeneration medium (modified Knop medium; 5% glucose; 3% mannitol; 540 mOsm; pH 5.6-5.8). Regeneration was performed as described by Strepp et al. (1998) Proc Natl Acad Sci USA 95, 4368-4373). Transgenic clones were identified by molecular screening.

### MALDI-Tof MS of moss glycans

Plant material was cultivated in liquid culture, isolated by filtration, frozen in liquid nitrogen and stored at -80 °C. The material was shipped under dry ice. The MALDI-TOF MS analyses were done in the laboratory of Prof. Dr. F. Altmann, Glycobiology Division, Institut für Chemie, Universität für Bodenkultur, Vienna, Austria.

0.2 to 0.5 g fresh weight of transgenic *Physcomitrella patens* material was digested with pepsin. N-glycans were obtained from the digest as described by Wilson et al. (2001). Essentially, the glycans were released by treatment with peptide:N-glycosidase A and analysed by MALDI-TOF mass spectrometry on a DYNAMO (Thermo BioAnalysis, Santa Fe, NM).

### 1. Identification of β1,3-galactosyltransferase encoding genes

Although biological functionality β1,3-galactosyltransferases (β-1,3galT) from humans in respect to the elongation of N-glycan structures was not described the sequence of the β-1,3galT 2 (Acc.No: CAA75344) of humans was choosen as starting sequence.

Based on the seven conserved domains described by Hennet (2002 Cell. Mol. Life Sci. 59, 1081-1095) and in combination with the conserved amino acids described by Amado et al. (1998 J. Biol. Chem. 273, 12770-12778) a database screening was performed. Due to this strategy one sequence from *Arabidopsis thaliana* (Acc.No: NP174003) and one sequence from *Oryza sativa* (Acc.No: BAD17812) described as putative β1,3-galactosyltransferases were identified. Although for both species numerous protein sequences of putative β1,3-galactosyltransferases were listed in the public databases only these two showed similarities on the one hand for the seven conserved domains and on the other hand for several of the highly conserved additional amino acids. However, if compared to CAA75344 the overall identity was very low for both, in case of NP174003 it was 16%, in case of BAD17812 it was 17% (Fig. 1).
All three protein sequences were used for the screening of a non public "expressed sequence tag" (EST) database of *Physcomitrella patens.* An expressed sequence tag encoding a peptide sequence which comprised some similarities with the seven conserved domains of the β1,3-galactosyltransfrases was identified. This EST was used to design primers for cloning purposes and for further screening in regard of a beta 1,3-galactosyltransferase gene family of a database comprising genomic sequences of *Physcomitrella patens.*
The resulting sequences comprised two putative β1,3-galactosyltransferase genes including intron and exon sequences and the gene structures (β-1,3galT 1 corresponds to SEQ ID NO: 1 and SEQ ID NO:3 and β-1,3galT 2 corresponds to SEQ ID NO: 2 and SEQ ID NO: 4). The protein sequences predicted from the open reading frames (β1,3-GalT 1 (Fig. 2) and β1,3-GalT 2 (Fig. 3) comprised transmembrane domains, the seven conserved domains and numerous of the conserved amino acids (Fig. 1).

### 1.1 Cloning of the coding sequence of β1,3-galactosyltransferase 1 gene from Physcomitrella patens

Amplification of the nucleotide sequence encoding β1,3-galactosyltransferase from *Physcomitrella patens*
(SEQ ID NO: 1: 5'AGTTGTCGATTTGTTGTTTTTGATATGTAAGGCGGTTGCCTTCGCGCCGTGCTTGATTGTAATTGTAATTCAATCTGGAGTGTGAGATATATATATATATATATATATAGCGAGAGGGAGAGAGAAAGAGAGAGAGAGGGAGAGAGAAAGAGAGAGAGAGGGAGAGAGAGAGATGGCTTGTGTATGAGGGCCATGCGAGGAGGAGGCTGTGTTTGTTGCCCGAAGAGATGGGATGGTTTATGTGTAGTGCAGGGGTTGGATGTGAAGCACCTGTTTGAAGGAGTCTGCGAGAGTTTGAAATTCGGATTCAGAGTGCGGCGATCGATGGTGCAACGTTGTTAGCAGTGATTGTTTTCGCCAACAGAACTGACATCATTTGGATTTTTTTTACGCGTGGATGTGCCCTCTTTTTAAAAAATTTCCGCGTGGAANAGAGACGGGGGTTTGTAATGGAGGCAGGCTGTGGTCATCACCCCTAGTATAGCCTGTCAAGAGAGTTCAAATTCGGTAAT**ATG**AAGAGGGGGTCGAGACTACCGGATATGGCGTGTACAGGGCGGCAAAGAAATGATCTTATCCTAGTTGCAATTGTTTGCTTGTTTTTTATGGTGATATTCATCCCACCATATCTCCAAATGAACTCACTTCCGGACATTGATTCTC
CTGATTCGGACAAGAAATCATCAAGCTACTCGAAAAAAACCACTCTAGAAGCCAATAGTAAGGAGGAACGCCGTAGTCCGGGGAATACCACAGGCGACATTGTTTCTCTGGATGATGTGATAGATCGTGCCTGGTCTGCTGGTGCCAAAGCGTGGGAAGAACTGGAAACTGCGTTAAGAAATGGAGAAGGTGTCTCAAAGAATGTCAGTAATGCCACTGCAAATGCTGATCCGTGTCCAGCATCACTCTCTGCAGCAGGGAAAAAGTTAGACGAATTGGGTAAAGTCTTCCCCTTGCCCTGTGGTCTAATGTTTGGGTCAGCCATTACTCTGATTGGAAAGCCTCGAGAGGCTCACATGGAGTACAAACCGCCAATCGCCAGAGTTGGGGAAGGCGTCTCTCCATATGTCATGGTTTCCCAGTTCTTAGTAGAGTTACAAGGCTTAAAGGTGGTGAAAGGTGAAGATCCTCCTCGAATTCTACACTTGAATCCTCGACTTCGTGGTGATTGGAGCTGGAAACCCATCATTGAGCACAACACTTGTTATCGGAACCAGTGGGGTCCTGCCCACCGATGCGAGGGTTGGCAAGTGCCTGAATACGAAGAAACTGTTGACGGTCTTCCCAAGTGCGAGAAGTGGCTTCGAGATGATGGCAAGAAACCTGCTTCAACGCAAAAATCTTGGTGGCTTGGAAGATTAGTTGGTCGTTCTGACAAGGAGACGCTTGAATGGGAGTACCCATTATCTGAGGGTCGGGAGTTCGTTCTCACCATTCGAGCAGGTGTTGAAGGGTTTCATGTGACTATCGATGGTCGTCACATCAGCTCGTTTCCTTATCGTGTGGGTTACGCTGTGGAAGAAACAACGGGGATATTAGTAGCAGGAGACGTTGATGTGATGTCTATCACAGTGACATCCCTACCCTTAACACATCCTAGCTACTACCCTGAGTTAGTTTTGGAATCGGGGGACATTTGGAAGGCACCACCTGTCCCAGCTACCAAGATAGATTTATTTATTGGGATCATGTCCAGCAGTAACCATTTTGCAGAACGGATGGCAGTAAGGAAGACGTGGTTTCAATCTAAAGCTATTCAATCTTCGCAGGCCGTGGCTCGCTTCTTTGTAGCTCTGCATGCAAACAAGGATATCAATATGCAGTTGAAGAAGGAGGCAGACTATTATGGCGATATTATAATCCTGCCTTTCATCGACAGATATGATATAGTGGTTCTCAAGACCGTTGAAATTTGCAAGTTTGGGGTCCAGAATGTCACAGCTAAGTATATTATGAAGTGTGACGATGACACTTTTGTGAGGATTGATAGCGTTCTCGAAGAGATTCGAACTACTTCAATATCACAAGGCCTTTACATGGGTAGCATGAATGAGTTTCACAGGCCTCTTCGTTCTGGAAAGTGGGCCGTGACTGCCGAGGAATGGCCTGAGCGAATTTACCCAATATATGCTAATGGACCAGGATATATCCTGTCAGAGGATATTGTGCATTTCATTGTGGAGATGAATGAGAGAGGCAGTTTGCAGTTATTTAAGATGGAGGACGTCAGTGTTGGAATATGGGTACGCGAATATGCGAAGCAAGTGAAGCACGTTCAATACGAACATAGCATACGGTTTGCTCAAGCCGGTTGTATACCGAAATACTTGACAGCTCATTACCAATCGCCGCGTCAAATGCTGTGTCTGTGGGACAAGGTACTTGCTCATGACGATGGGAAATGCTGCAACTTG**TGA**GGAAAATACATACAATGAATGTGTTCAACGGTCTTTACCAGACAGAATTACTTTGGGTCGGGAACCAGATATAGCAGACAGCTCACATTCAATTCAGCCGTGTTGATCCAGAGGGGTAATTGATAGTTTCCTTGTCCCCTACCCTCTCTAGAGGTGGAGATCTTACAACTTAATCAAATGATCCTCTGCAATGTCACTTGTCACAATACTTAGTATAGCTCAAAATTGGCCACGGATATTCAGGAATGTTCATCTTGTAAGGTCGCAGCTTGTGAGTAAATGGTTGGGTGGTGTCGATGGCATGGTTGCTTATCAATCCCTCTTAGCATCAGTGATCGTCAGAATCAGTGTTTTCGACACTCCCCGGTGGAGTATTTTTTCGATTCTCTTGATTCCACTCAAGTGGTACTAGCTTATATTTAGTGAGGCCTGGAACCCAAGTAGTTAGTTCAGTACGTCTGCCTTTTGCCGAAATGAGTAGAGTAATTTGTGGCAGTAGTTGGTGAAGAGACATGGTTAGGATTTAGTGTTCAAAATCTG 3'; start and stop codon are indicated in bold letters) was performed by PCR with cDNA and the primers MOB1251, (SEQ ID NO: 5: 5'- CTGAATATCCGTGGCCAA -3') and primer MOB 1410 (SEQ ID NO: 6: 5'- TTCGAGCTCATGAAGAGGGGGTCGAGACT -3'). The amplification product was digested with Sac I and Msc I and cloned into the Sac I/Sma I digested vector pRT101 (Toepfer et al. 1987 NAR 15, 5890). The cloned sequence was verified by sequencing.

### 1.2 Cloning of the coding sequence of β1,3-galactosyltransferase 2 gene from Physcomitrella patens

Amplification of the nucleotide sequence encoding β1,3-galactosyltransferase from *Physcomitrella patens* (SEQ ID NO: 2: 5'-**ATG**AAGAGGGGTGTGAGACCACCGGGTGTGGGATGTACAGGGCGGCAAAGAAACAATCTAATCATAGTGGCAATCATATGTTTGGTTTTTATAGCGATATTCATCCCACCGTTTCTTGAAATGAATTCACTTCCCGATATTGATTCCCCTGTTTTGGAGAAGAAAGTATCAAGCTATTTGAAAAAAGTCACTCTGGAAACTTACAGTAAAGAGGAACGCCGTAGTCCAGGGAACACAACAGGTGACATTGTTTCGCTGGAAGATGTGATAGATCGCGCCTGGTCTGCCGGCGCCAAAGCTTGGGAAGAGCTGGAAATTGCATTCAGACAGGGAGAACATTTTTCGAAGAAGGACAATAATGCCAATGCAACTGCAGATCCATGCCCAGCATCACTCTTTACAACAGGAAAGGAATTGGACAATTTAGGAAGGGTCTTCCCACTGCCTTGTGGTCTAATGTTTGGATCAGCCATAACTCTCATTGGAAAGCCACGGGAAGCTCACATGGAGTACAAACCGCCAATCGCCAGAGTTGGGGAAGGTGTCTCTCCATACGTCATGGTGTCCCAGTTCATAATGGAGTTACAGGGCTTGAAGGTG
GTAAAAGGTGAAGATCCTCCTAGAATCCTCCACATAAACCCTCGACTCCGTGGTGACTGGAGCTGGAAACCCATCATTGAGCATAATACATGCTATCGAAACCAGTGGGGCCCAGCTCATCGGTGTGAAGGTTGGCAAGTACCTGAATACGAAGAAACCGTGGACGGTCTTCCCAAGTGCGAGAAGTGGCTTCGAGGCGATGACAAAAAACCTGCTTCGACCCAAAAATCCTGGTGGCTTGGGCGATTAGTTGGTCATTCCGACAAGGAGACGCTTGAATGGGAGTATCCATTGTCCGAAGGTCGGGAGTTTGTTCTCACCATTCGAGCAGGTGTAGAAGGATTTCACTTAACTATTGATGGTCGGCACATCAGTTCGTTCCCTTATCGTGCGGGTTATGCTATGGAAGAAGCAACAGGAATATCAGTGGCAGGAGACGTCGATGTTCTTTCGATGACAGTAACATCATTACCTTTAACACATCCCAGCTACTACCCTGAGTTGGTTTTGGATTCGGGTGATATCTGGAAGGCACCACCTTTACCAACAGGCAAGATAGAGTTATTTGTTGGAATCATGTCAAGCAGCAATCACTTTGCAGAACGTATGGCAGTAAGAAAGACGTGGTTTCAGTCTCTGGTTATCCAATCCTCCCAAGCGGTGGCTCGCTTCTTTGTAGCTCTGCATGCAAACAAGGATATCAATCTGCAGCTGAAGAAAGAGGCTGACTATTACGGCGATATGATAATTTTACCTTTCATCGACAGATATGATATAGTGGTTCTTAAGACCGTTGAAATTTTCAAGTTTGGGGTCCAGAATGTTACAGTTAGCCACGTCATGAAATGTGACGATGACACATTTGTAAGGATTGACAGCGTTCTTGAAGAGATTCGAACGACGTCAGTAGGACAGGGCCTTTACATGGGCAGCATGAATGAGTTTCATAGACCCCTTCGTTCTGGGAAGTGGGCCGTGACAGTTGAGGAGTGGCCTGAGCGCATTTACCCAACATACGCAAATGGTCCAGGATACATCCTTTCGGAAGATATTGTGCATTTTATAGTGGAGGAGAGCAAAAGAAATAATTTGAGGTTATTTAAGATGGAGGACGTCAGCGTAGGTATATGGGTACGCGAGTATGCAAAGATGAAGTACGTGCAATACGAGCATAGCGTACGGTTTGCTCAAGCCGGTTGTATACCTAACTACCTGACAGCGCACTATCAATCGCCGCGTCAAATGCTGTGTCTGTGGGACAAGGTGCTTGCTACCAATGACGGCAAGTGCTGCACCTTG**TGA** -3'; start and stop codon are indicated in bold letters) was performed by PCR with cDNA and the primers Ppβ1-3 GalT2 for (SEQ ID NO: 7: 5'- TACGAGCTCATGAAGAGGGGTGTGAGACC -3') and primer Ppβ1-3GalT2 rev (SEQ ID NO: 8: 5'-GTAGAGCTCTCACAAGGTGCAGCACTTG -3'). The amplification product was digested with Sac I and cloned into the Sac I digested vector pRT101 (Toepfer et al. 1987 NAR 15, 5890). The cloned sequence was verified by sequencing.

### 2.1 Creating the knockout construct of the β1,3-galactosyltransferase 1 gene from Physcomitrella patens

The knockout construct for targeted gene disruption of the β1,3-galactosyltransferase 1 gene of *Physcomitrella patens* was generated by PCR performed with genomic DNA from *Physcomitrella patens.* In one PCR primer MOB1336 (SEQ ID NO: 9: 5'- TACGGATCCAACTTCGAGTTCGTGTCTGTA -3') and primer MOB1333 (SEQ ID NO: 10: 5'- ACACT**AAGCTT**CTAATCAATGTCCGGAAGTGAG -3') were used to amplify the 5' part of the knockout construct. In a second PCR primer MOB1334 (SEQ ID NO: 11: 5'- TTAG**AAGCTT**AGTGTACGCTGAGTGTCTACATTG -3') and primer MOB1335 (SEQ ID NO: 12: 5'- CATTGTCGACCCTACACAGCTCTTAACGTCTAC -3') were used to amplify the 3' part of the knockout construct. Both amplified constructs were digested with Hin dIII (restriction sites are indicated in the primer sequences MOB1333 and MOB1334 in bold letters) and were ligated in a subsequent ligation reaction using T4 DNA ligase. The resulting ligated and purified DNA sequence was used as template for a further PCR with primer MOB1336 and MOB1335. The resulting amplification product β1-3GalT1ko (SEQ ID NO: 13: 5'- CAACTTCGAGTTCGTGTCTGTATGAAGAAGTCCACGGGTTCAATGTGTTAAGACTTAGGCATTTCCTTCAGCTTTGCCTAGTGGAGATATGCGTATTTTTTGATTGTGAGGATTCCGGTTCTTAGACCATGATTGGTTTATTACAGTGGTCATTCAAATCCTATTTGATTTGAGAATGTATTTACTTCGTTGTGTTGGGAGATGATTGTTCCCTCGAATTCTATGCGGTAGCTACCGCTTCTTTCGTAATGAAGACCTTTGAAGTTCACATAGACTTCAAGAAGAATGCTATTTGTGTTTTTGTGATTGTGTGTTCAAGTTTGGTGCAGTATTGTTAAAATTTGGGTGATGACTAAGTACACTTTATGCGGCCCAAGTAGTCAAGTTGAGCATTTGTAAATGCTGAAATGAGTTAGGCTGACGGTAAATGTCTGTGGATGTAGCCTAGTGATGTATTTGATCTCGGCATAATCTTCAGTGATCAATACAAATAATTCAAGAAAGAGGGGTCAATGTGTTCCTGCGAGTACCTTCGCATGTTCAACGTGAACTGAATTATGTTAATTAAGCTGAGCAACATAGACCTTCTTGCTGTTGACAGAGTTCAAATTCGGTAATATGAAGAGGGGGTCGAGACTACCGGATATGGCGTGTACAGGGCGGCAAAGAAATGATCTTATCCTAGTTGCAATTGTTTGCTTGTTTTTTATGGTGATATTCATCCCACCATATCTCCAAATGAACTCACTTCCGGACATTGATTAG**AAGCTT**AGTGTACGCTGAGTGTCTACATTGTGTATTGAATGTTCCTTAGAATTGTTTGTTTGTTTATGTTTTTATTTTTATATTTCTGCCGGCTATTGAGGAAGAATACATTCAAATTGTTCAGGATTCGGACAAGAAATCATCAAGCTACTCGAAAAAAACCACTCTAGAAGCCAATAGTAAGGAGGAACGCCGTAGTCCGGGGAATACCACAGGCGACATTGTTTCTCTGGATGATGTGATAGATCGTGCCTGGTCTGCTGGTGCCAAAGCGTGGGAAGAACTGGAAACTGCGTTAAGAAATGGAGAAGGTGTCTCAAAGAATGTCAGTAATGCCACTGCAAATGCTGATCCGTGTCCAGCATCACTCTCTGCAGCAGGGAAAAAGTTAGACGAATTGGGTAAAGTCTTCCCCTTGCCCTGTGGTCTAATGTTTGGGTCAGCCATTACTCTGATTGGAAAGCCTCGAGAGGCTCACATGGAGTACAAACCGCCAATCGCCAGAGTTGGGGAAGGCGTCTCTCCATATGTCATGGTTTCCCAGTTCTTAGTAGAGTTACAAGGCTTAAAGGTGGTGAAAGGTGAAGATCCTCCTCGAATTCTACACTTGAATCCTCGACTTCGTGGTGATTGGAGCTGGAAACCCATCATTGAGCACAACACTTGTTATCGGAACCAGTGGGGTCCTGCCCACCGATGCGAGGGTTGGCAAGTGCCTGAATACGAAGAAACTGGTGAGTGCTGATTCCACCGCACCAGTTTGTGTTTTTTATGCTGACACTATGCTTCTCAGGTTTGTAGACGTTAAGAGCTGTGTAGG -3'; Hin dIII restriction site is indicated in bold letters) comprised a deletion of 270 bp in regard to the genomic sequence of the β1,3-galactosyltransferase gene 1 of *Physcomitrella patens* which in addition initiate a stop codon in the early 5' part of the corresponding cDNA. Thus, resulting in a dyscfunctional β1,3-galactosyltransferase gene when integrated via homologous recombination into the genome of *Physcomitrella patens.* This knockout construct was used for transformation of Phy*scomitrella patens* alone or in combination with knockout construct β1-3GalT2ko (see 2.2).

Screening of putative transformed plants was performed by PCR using appropriate primer combinations.

### 2.2 Creating the knockout construct of the β1,3-galactosyltransferase 2 gene from Physcomitrella patens

The knockout construct for targeted gene disruption of the β1,3-galactosyltransferase 2 gene of *Physcomitrella patens* was generated by PCR performed with genomic DNA from *Physcomitrella patens.* In one PCR primer MOB1339 (SEQ ID NO: 14: 5'- TGGCACGATACAGTGGCATGA -3') and primer MOB1337 (SEQ ID NO: 15: 5'-TG**GAATTC**ATTCAAGAAACGGTGGGATGA -3') were used to amplify the 5' part of the knockout construct. In a second PCR primer MOB1338 (SEQ ID NO: 16: 5'- T**GAATTC**CATAACGAAGACACCGTCTA -3') and primer MOB1313 (SEQ ID NO: 17: 5'- CAAGCAGCGGAGACCTTGCAATGC -3') were used to amplify the 3' part of the knockout construct. Both amplified constructs were digested with Eco RI (restriction sites are indicated in the primer sequences MOB1337 and MOB1338 in bold letters) and were ligated in a subsequent ligation reaction using T4 DNA ligase. The resulting ligated and purified DNA sequence was used as template for a further PCR with primer MOB1339 and MOB1313. The resulting amplification product β1-3GalT2ko (SEQ ID NO: 18: 5'- TGGCACGATACAGTGGCATGAGATTTATCGCTGCCAAACTGTGGACAATGATGTTTGAAACAGTCTATTCATCACTGGTTGGCAAATTCTATGTACAGGGCTAAAAGGGCCAAACTAGGCTTAACAGCAGTGATCGAGGTTCTTGAGCAGGATCAGCGCAAGGGTAAGGTTGCTTAGGACCGCTTCAACCTGGTGAGTTAGACACTCAAAATAATTACGAAACAGTGACATTTATAAGCTTTGTGTCGTCACTACTTTGAGCCTTCAGAGTACATTTATAGGTGGTGACTTCGTTAATGATGTTAAAAATATGAGGTGAGGACATGTCTTCTTGTGATTAGAGTGATCACTTTGATCCTTTTGCAAACGCTGAAAGGAGTAAGTCTGATTGTCAACAGAAATGTTTTTGGTTGCAGCCTGGCTAATATTATTGGTCTCAGTTCAATTTTCGATGGAGTGGCGTACAAGTGATCCAGAAAGCAAGAATCATGGATTTCCTACAATTTCATTTAGATTTTCGATGTTGGTTGAGTTATGCTGATTGATTTGGGAAAGAGGGAGCTTAGCGTTGTATACAGGGTTCAAACACCGTAATATGAAGAGGGGTGTGAGACCACCGGGTGTGGGATGTACAGGGCGGCAAAGAAACAATCTAATCAT
AGTGGCAATCATATGTTTGGTTTTTATAGCGATATTCATCCCACCGTTTCTTGAAT**GAATTC**CATAACGAAGACACCGTCTAAAGCTTCACAGGTTAGTGCAGAAATGATTGGTTCGCCCTCGCTATGCCAGTCAGGCTTACTGAGTTCTACTTGGATCGTTCTACTTGGATCTTTTATGGCTTCCTAGCAGTCGGAGGTTTCTTTCTGGTTTGAAGAAAGCCATGTATGGAACGTTTACAGGTTTTGGAGAAGAAAGTATCAAGCTATTTGAAAAAAGTCACTCTGGAAACTTACAGTAAAGAGGAACGCCGTAGTCCAGGGAACACAACAGGTGACATTGTTTCGCTGGAAGATGTGATAGATCGCGCCTGGTCTGCCGGCGCCAAAGCTTGGGAAGAGCTGGAAATTGCATTCAGACAGGGAGAACATTTTTCGAAGAAGGACAATAATGCCAATGCAACTGCAGATCCATGCCCAGCATCACTCTTTACAACAGGAAAGGAATTGGACAATTTAGGAAGGGTCTTCCCACTGCCTTGTGGTCTAATGTTTGGATCAGCCATAACTCTCATTGGAAAGCCACGGGAAGCTCACATGGAGTACAAACCGCCAATCGCCAGAGTTGGGGAAGGTGTCTCTCCATACGTCATGGTGTCCCAGTTCATAATGGAGTTACAGGGCTTGAAGGTGGTAAAAGGTGAAGATCCTCCTAGAATCCTCCACATAAACCCTCGACTCCGTGGTGACTGGAGCTGGAAACCCATCATTGAGCATAATACATGCTATCGAAACCAGTGGGGCCCAGCTCATCGGTGTGAAGGTTGGCAAGTACCTGAATACGAAGAAACCGGTGAGTGCTGGTTCCATCACACTTTATCTTTTCATAGTGACACGGTTCTTTTTAGGTGTACTAGTGTTGAAAGCTGTGCATGTTAAATGGTAACCCTAATCAATCTTCTCGCTAATTTTCGCATTGCAAGGTCTCCGCTGCTTG -3'; Eco RI restriction site is indicated in bold letters) comprised a deletion of 148 bp in regard to the genomic sequence of the β1,3-galactosyltransferase 2 gene of *Physcomitrella patens* which in addition initiate a stop codon in the early 5' part of the corresponding cDNA. Thus, resulting in a dyscfunctional β1,3-galactosyltransferase gene when integrated via homologous recombination into the genome of *Physcomitrella patens.* This knockout construct was used for transformation of *Physcomitrella patens* alone or in combination with the knockout construct β1-3GalT1ko (see 2.1).

Screening of putative transformed plants was performed by PCR using appropriate primer combinations.

### 3. MALDI-TOF mass spectrometry

The N-glycans of glyco-engineered *Physcomitrella patens* strain lacking plant-specific core α1,3 fucose and β1,2 xylose residues - herein used as control - exhibit the typical structural features of plant N-glycans processed in these strains as described in Koprivova et al. 2004 Plant Biotechnol. J. 2, 517-523); i.e. no fucose in α1,3-linkage to the Asn-bound GlcNAc, and no xylose in β1,2-linkage to the βmannosyl reidue, Lewis a epitopes (α1,4-fucosyl and β1,3-galactosyl residues linked to GlcNAc) as non reducing terminal elements (tab. 1). In contrast no Lewis a epitopes (α1,4-fucosyl and β1,3-galactosyl residues linked to GlcNAc) were detected on N-glycans isolated from a glyco-engineered *Physcomitrella patens* strain which additionally comprised targeted gene disruptions of both β1,3-galactosyltransferase 1 and β1,3-galactosyltransferase 2 genes.

**Tab.1: N-glycan structures of double knockout and tetra knockout Physcomitrella patens strains. N-glycans were isolated from plant material grown under same conditions (100 ml flasks, Knop medium) residues, GF = Lewis a structure comprising fucose and galactose (β1,3-linked), Gn = N-acetylglucosamine, M/Man = mannose**

| | Physcomitrella patens double knockout | Physcomitrella patens tetra knockout |
|---|---|---|
| | N-glycan structures lacking core α1,3-fucose and β1,2-xylose residues | N-glycan structures lacking core α1,3-fucose, β1,2-xylose and β1,3-galactose residues (consequently lacking Lewis a epitopes in total) |
| 933 | Man3 (MM) | Man3 (MM) |
| 1096 | Man4 | Man4 |
| 1137 | MGn/GnM | MGn/GnM |
| 1258 | Man5 | Man5 |
| 1299 | Man4Gn | Man4Gn |
| 1340 | GnGn | GnGn |
| 1420 | Man6 | Man6 |
| 1582 | Man7 | Man7 |
| 1648 | (GF)Gn/Gn(GF) | |
| 1744 | Man8 | Man8 |
| 1907 | Man9 | Man9 |
| 1956 | (GF) (GF) | |

SEQ ID NO: 1

cDNA β1-3GalT1

5´AGTTGTCGATTTGTTGTTTTTGATATGTAAGGCGGTTGCCTTCGCGCCGTGCTTGATTGTAAT-
TGTAATTCAATCTGGAGTGTGAGATATATATATATATATATATATAGCGAGAGGGAGAGAGAAAGAGAGAGAGAGG-
GAGAGAGAAAGAGAGAGAGAGGGAGAGAGAGAGATGGCTTGTGTATGAGGGCCATGCGAGGAGGAGGCTGT-
GTTTGTTGCCCGAAGAGATGGGATGGTTTATGTGTAGTGCAGGGGTTGGATGTGAAGCACCTGTTTGAAGGAGTCT-
GCGAGAGTTTGAAATTCGGATTCAGAGTGCGGCGATCGATGGTGCAACGTTGTTAGCAGTGATTGTTTTCGC-
CAACAGAACTGACATCATTTGGATTTTTTTACGCGTGGATGTGCCCTCTTTTTAAAAAATTTCCGCGTGGAANA-
GAGACGGGGGTTTGTAATGGAGGCAGGCTGTGGTCATCACCCCTAGTATAGCCTGTCAAGAGAGTTCAAATTCG-
GTAATATGAAGAGGGGGTCGAGACTACCGGATATGGCGTGTACAGGGCGGCAAAGAAATGATCTTATCCTAGT-
TGCAATTGTTTGCTTGTTTTTATGGTGATATTCATCCCACCATATCTCCAAATGAACTCACTTCCGGACAT-
TGATTCTC
CTGATTCGGACAAGAAATCATCAAGCTACTCGAAAAAAACCACTCTAGAAGCCAATAGTAAGGAGGAACGC-
CGTAGTCCGGGGAATACCACAGGCGACATTGTTTCTCTGGATGATGTGATAGATCGTGCCTGGTCTGCTGGTGC-
CAAAGCGTGGGAAGAACTGGAAACTGCGTTAAGAAATGGAGAAGGTGTCTCAAAGAATGTCAGTAATGCCACT-
GCAAATGCTGATCCGTGTCCAGCATCACTCTCTGCAGCAGGGAAAAAGTTAGACGAATTGGGTAAAGTCTTCCCCT-
TGCCCTGTGGTCTAATGTTTGGGTCAGCCATTACTCTGATTGGAAAGCCTCGAGAGGCTCACATGGAGTACAAAC-
CGCCAATCGCCAGAGTTGGGGAAGGCGTCTCTCCATATGTCATGGTTTCCCAGTTCTTAGTAGAGTTACAAGGCT-
TAAAGGTGGTGAAAGGTGAAGATCCTCCTCGAATTCTACACTTGAATCCTCGACTTCGTGGTGATTGGAGCTG-
GAAACCCATCATTGAGCACAACACTTGTTATCGGAACCAGTGGGGTCCTGCCCACCGATGCGAGGGTTGGCAAGT-
GCCTGAATACGAAGAAACTGTTGACGGTCTTCCCAAGTGCGAGAAGTGGCTTCGAGATGATGGCAAGAAACCT-
GCTTCAACGCAAAAATCTTGGTGGCTTGGAAGATTAGTTGGTCGTTCTGACAAGGAGACGCTTGAATGGGAGTAC-
CCATTATCTGAGGGTCGGGAGTTCGTTCTCACCATTCGAGCAGGTGTTGAAGGGTTTCATGTGACTATCGATG-
GTCGTCACATCAGCTCGTTTCCTTATCGTGTGGGTTACGCTGTGGAAGAAACAACGGGGATATTAGTAGCAG-
GAGACGTTGATGTGATGTCTATCACAGTGACATCCCTACCCTTAACACATCCTAGCTACTACCCTGAGT-
TAGTTTTGGAATCGGGGGACATTTGGAAGGCACCACCTGTCCCAGCTACCAAGATAGATTTATTTATTGGGATCAT-
GTCCAGCAGTAACCATTTTGCAGAACGGATGGCAGTAAGGAAGACGTG-
GTTTCAATCTAAAGCTATTCAATCTTCGCAGGCCGTGGCTCGCTTCTTTGTAGCTCTGCATGCAAACAAGGATA-
TCAATATGCAGTTGAAGAAGGAGGCAGACTATTATGGCGATATTATAATCCTGCCTTTCATCGACAGATATGATA-
TAGTGGTTCTCAAGACCGTTGAAATTTGCAAGTTTGGGGTCCAGAATGTCACAGCTAAGTATATTATGAAGTGT-
GACGATGACACTTTTGTGAGGATTGATAGCGTTCTCGAAGAGATTCGAACTACTTCAATATCACAAGGCCTTTA-
CATGGGTAGCATGAATGAGTTTCACAGGCCTCTTCGTTCTGGAAAGTGGGCCGTGACTGCCGAGGAATGGCCT-
GAGCGAATTTACCCAATATATGCTAATGGACCAGGATATATCCTGTCAGAGGATATTGTGCATTTCATTGTGGAG-
ATGAATGAGAGAGGCAGTTTGCAGTTATTTAAGATGGAGGACGTCAGTGTTGGAATATGGGTACGCGAATA-
TGCGAAGCAAGTGAAGCACGTTCAATACGAACATAGCATACGGTTTGCTCAAGCCGGTTGTATACCGAAATACT-
TGACAGCTCATTACCAATCGCCGCGTCAAATGCTGTGTCTGTGGGACAAGGTACTTGCTCATGACGATGGAAAT-
GCTGCAACTTGTGAGGAAAATACATACAATGAATGTGTTCAACGGTCTTTACCAGACAGAATTACTTTGGGTCGG-

GAACCAGATATAGCAGACAGCTCACATTCAATTCAGCCGTGTTGATCCAGAGGGGTAATTGATAGTTTCCT-
TGTCCCCTACCCTCTCTAGAGGTGGAGATCTTACAACTTAATCAAATGATCCTCTGCAATGTCACTTGT-
CACAATACTTAGTATAGCTCAAAATTGGCCACGGATATTCAGGAATGTTCATCTTGTAAGGTCGCAGCTTGT-
GAGTAAATGGTTGGGTGGTGTCGATGGCATGGTTGCTTATCAATCCCTCTTAGCATCAGTGATCGTCAGAATCAGT-
GTTTTCGACACTCCCCGGTGGAGTATTTTTTCGATTCTCTTGATTCCACTCAAGTGGTACTAGCTTATATTTAGT-
GAGGCCTGGAACCCAAGTAGTTAGTTCAGTACGTCTGCCTTTTGCCGAAATGAGTAGAGTAATTTGTGGCAGTAGT-
TGGTGAAGAGACATGGTTAGGATTTAGTGTTCAAAATCTG 3´

SEQ ID NO: 2

cDNA Ppβ1-3GalT2

ATGAAGAGGGGTGTGAGACCACCGGGTGTGGGATGTACAGGGCGGCAAAGAAACAATCTAAT-
CATAGTGGCAATCATATGTTTGGTTTTTATAGCGATATTCATCCCACCGTTTCTTGAAAT-
GAATTCACTTCCCGATATTGATTCCCCTGTTTTGGAGAAGAAAGTAT-
CAAGCTATTTGAAAAAAGTCACTCTGGAAACTTACAGTAAAGAGGAACGCCGTAGTCCAGG-
GAACACAACAGGTGACATTGTTTCGCTGGAAGATGTGATAGATCGCGCCTGGTCTGCCGGCGC-
CAAAGCTTGGGAAGAGCTGGAAATTGCATTCAGACAGGGAGAACATTTTTCGAAGAAG-
GACAATAATGCCAATGCAACTGCAGATCCATGCCCAGCATCACTCTTTACAACAGGAAAG-
GAATTGGACAATTTAGGAAGGGTCTTCCCACTGCCTTGTGGTCTAATGTTTGGATCAGC-
CATAACTCTCATTGGAAAGCCACGGGAAGCTCACATGGAGTACAAACCGCCAATCGCCAGAGT-
TGGGGAAGGTGTCTCTCCATACGTCATGGTGTCCCAGTTCATAATGGAGTTACAGGGCT-
TGAAGGTG
GTAAAAGGTGAAGATCCTCCTAGAATCCTCCACATAAACCCTCGACTCCGTGGTGACTG-
GAGCTGGAAACCCATCATTGAGCATAATACATGCTATCGAAACCAGTGGGGCCCAGCTCATCG-
GTGTGAAGGTTGGCAAGTACCTGAATACGAAGAAACCGTGGACGGTCTTCCCAAGTGC-
GAGAAGTGGCTTCGAGGCGATGACAAAAAACCTGCTTCGACCCAAAAATCCTGGTGGCTTGG-
GCGATTAGTTGGTCATTCCGACAAGGAGACGCTTGAATGGGAGTATCCATTGTCCGAAG-
GTCGGGAGTTTGTTCTCACCATTCGAGCAGGTGTAGAAGGATTTCACTTAACTATTGATG-
GTCGGCACATCAGTTCGTTCCCTTATCGTGCGGGTTATGCTATGGAAGAAGCAACAGGAATA-
TCAGTGGCAGGAGACGTCGATGTTCTTTCGATGACAGTAACATCATTACCTTTAACA-
CATCCCAGCTACTACCCTGAGTTGGTTTTGGATTCGGGTGATATCTGGAAGGCAC-
CACCTTTACCAACAGGCAAGATAGAGTTATTTGTTGGAATCATGTCAAGCAGCAAT-
CACTTTGCAGAACGTATGGCAGTAAGAAAGACGTGGTTTCAGTCTCTGGT-
TATCCAATCCTCCCAAGCGGTGGCTCGCTTCTTTGTAGCTCTGCATGCAAACAAGGATA-
TCAATCTGCAGCTGAAGAAAGAGGCTGACTATTACGGCGATA-
TGATAATTTTACCTTTCATCGACAGATATGATATAGTGGTTCTTAAGACCGT-
TGAAATTTTCAAGTTTGGGGTCCAGAATGTTACAGTTAGCCACGTCATGAAATGTGACGAT-
GACACATTTGTAAGGATTGACAGCGTTCTTGAAGAGATTCGAACGACGTCAGTAGGACAGG-

GCCTTTACATGGGCAGCATGAATGAGTTTCATAGACCCCTTCGTTCTGGGAAGTGGGCCGT-
GACAGTTGAGGAGTGGCCTGAGCGCATTTACCCAACATACGCAAATGGTCCAGGATA-
CATCCTTTCGGAAGATATTGTGCATTTTATAGTGGAGGAGAGCAAAAGAAATAATTTGAGGT-
TATTTAAGATGGAGGACGTCAGCGTAGGTATATGGGTACGCGAGTATGCAAAGAT-
GAAGTACGTGCAATACGAGCATAGCGTACGGTTTGCTCAAGCCGGTTGTATACCTAACTACCT-
GACAGCGCACTATCAATCGCCGCGTCAAATGCTGTGTCTGTGGACAAGGTGCTTGCTAC-
CAATGACGGCAAGTGCTGCACCTTGTGA

SEQ ID NO: 3

Genomic DNA β1-3GalT1

5´:AGTTGTCGATTTGTTGTTTTTGATATGTAAGGCGGTTGCCTTCGCGCCGTGCTTGATTGTAAT-
TGTAATTCAATCTGGAGTGTGAGATATATATATATATATATATATAGCGAGAGGGAGAGAGAAAGAGAGAGAGAGG-
GAGAGAGAAAGAGAGAGAGAGGGAGAGAGAGAGATGGCTTGTGTATGAGGGCCATGCGAGGAGGAGGCTGT-
GTTTGTTGCCCGAAGAGATGGGATGGTTTATGTGTAGTGCAGGGGTTGGATGTGAAGCACCTGTTTGAAGGAGTCT-
GCGAGAGTTTGAAATTCGGATTCAGAGTGCGGCGATCGATGGTGCAACGTTGTTAGCAGTGATTGTTTTCGC-
CAACAGAACTGACATgtaatgaatagtttcgaggcatgatcgcggtttttctcaatttgaaggggttgtttgtgg-
gtgatctatgtgcagaagtgtcactgatggtcagattcgatgcttgacaatttgatcctttgtgagtgtgcagC-
ATTTGGATTTTTTTTACGCGTGGATGTGCCCTCTTTTTAAAAAATTTCCGCGTGGAAAAGAGACGGGG-
GTTTGTAATGGAGGCAGGCTGTGGTCATCACCCCTAGTATAGCCTGTCAAGAGgtgagattgacaccctctttgct-
caattgtagatttttttccttctcagggct-
gaatcccagtttttttttttttttttttttttttccttcttcttcaacttcgagttcgtgtctgtat-
gaagaagtccacgggttcaatgtgttaagacttaggcatttccttcagctttgcctagtggagata-
tgcgtatttttttgattgtgaggattccggttcttagaccatgattggtttattacagtggt-
cattcaaatcctatttgatttgagaatgtatttacttcgttgtgttgggagatgattgttccctcgaattctat-
gcggtagctaccgcttctttcgtaatgaagacctttgaagttcacatagacttcaagaagaatgctatttgt-
gtttttgtgattgtgtgttcaagtttggtgcagtattgttaaaatttgggtgatgactaagtacactttatgcggc-
ccaagtagtcaagttgagcatttgtaaatgctgaaatgagttaggctgacggtaaatgtctgtggatgtagccta-
gtgatgtatttgatctcggcataatcttcagtgatcaatacaaataattcaagaaagaggggtcaatgtgttcct-
gcgagtaccttcgcatgttcaacgtgaactgaattatgttaattaagctgagcaacatagaccttcttgctgt-
tgacagAGTTCAAATTCGGTAATATGAAGAGGGGGTCGAGACTACCGGATATGGCGTGTACAGGGCG-
GCAAAGAAATGATCTTATCCTAGTTGCAATTGTTTGCTTGTTTTTATGGTGATATTCATCCCACCATA-
TCTCCAAATGAACTCACTTCCGGACATTGATTCTCCTgtcgagaagctagaagatgatgatgatgct-
gtcttcacttctcatagacgtcgtaaccaagagcagatttcagttgtcactgacagtggtcagagacggacagt-
tatgccatcttcgactggtgcggaggacgtaacgaatgcaccgtctaaagattcacaggttagaccaaaagtagt-
tgacctgaaatgcatgtggtaatcaagcactcttgtccttattcgagcttttatttcttgccatcag-

gtatttttaatacttccctagtgtacgctgagtgtctacattgtgtattgaatgttccttagaat-
tgtttgtttgtttatgttttatttttatatttctgccggctattgaggaagaatacattcaaattgttcag-
GATTCGGACAAGAAATCATCAAGCTACTCGAAAAAAACCACTCTAGAAGCCAATAGTAAGGAGGAACGC-
CGTAGTCCGGGGAATACCACAGGCGACATTGTTTCTCTGGATGATGTGATAGATCGTGCCTGGTCTGCTGGTGC-
CAAAGCGTGGGAAGAACTGGAAACTGCGTTAAGAAATGGAGAAGGTGTCTCAAAGAATGTCAGTAATGCCACT-
GCAAATGCTGATCCGTGTCCAGCATCACTCTCTGCAGCAGGGAAAAAGTTAGACGAATTGGGTAAAGTCTTCCCCT-
TGCCCTGTGGTCTAATGTTTGGGTCAGCCATTACTCTGATTGGAAAGCCTCGAGAGGCTCACATGGAGTACAAAC-
CGCCAATCGCCAGAGTTGGGGAAGGCGTCTCTCCATATGTCATGGTTTCCCAGTTCTTAGTAGAGTTACAAGGCT-
TAAAGGTGGTGAAAGGTGAAGATCCTCCTCGAATTCTACACTTGAATCCTCGACTTCGTGGTGATTGGAGCTG-
GAAACCCATCATTGAGCACAACACTTGTTATCGGAACCAGTGGGGTCCTGCCCACCGATGCGAGGGTTGGCAAGT-
GCCTGAATACGAAGAAACTGgtgagtgctgattccaccgcaccagtttgtgtttttatgctgacactatgcttct-
caggtttgtagacgttaagagctgtgtaggttccgtggtacttcgaattggcacttgccacttctctcat-
tgtaagttggtaaatgtctgcatgagcaataaattccaacactggatgtgtattttctgaaatgattcgttttct-
tgtagTTGACGGTCTTCCCAAGTGCGAGAAGTGGCTTCGAGATGATGGCAAGAAACCTGCTTCAACGCAAAAATCT-
TGGTGGCTTGGAAGATTAGTTGGTCGTTCTGACAAGGAGACGCTTGAATGGGAGTACCCATTATCTGAGGGTCGG-
GAGTTCGTTCTCACCATTCGAGCAGGTGTTGAAGGGTTTCATGTGACTATCGATGGTCGTCACAT-
CAGCTCGTTTCCTTATCGTGTGgtaagttgaaaatgctatgttaacatataatgctaaagttgacctcat-
gtctttctttttctttttttctttttatttctggaggggggggggtaatgcaaat-
caactctaaaatttagtataccagttaaattattcatttcaaatataacaatacaaataca-
catctttttaatttgtatttttgatccctctcctcctctactaaaattaataatatagcaacattttggtac-
tacgaaagttcatttgtattgcttcatgtcgaagatttattcaaatttctatccctcgtgtttctgaattacat-
tatcaacaatggaataacaataatgacggccccatccttcagacaccaggaacattacctataccagactacgtct-
gggtaagtctgaagaattaattataaccaagaaactagttgtattcactgtttttcttttttacgcccat-
gcgatttatcgaagtcttcttcaatttcttattattcttctttattattttaagtttttaat-
tatttttaaagcaacgaattgataaataaataacatattaat-
gtttttaactttaaagtttttttcccgtatttagtataagatttcgtcaaaacgattaggtgattagatcgaacat-
tatctaattgcactctacttatatgatatgaagagtaatttctcttagcagaagctacatcctgctatttccttgg-
gaaacccgattaggtctttcaaatcacccctgcttcctctataagtgtaccatgattgaggttcgttagggc-
attagtttaagggtatcgttgtgatgtgtgtctagttagtcttaaaatctgtgcaaatcgattcat-
taacaactcttttctgtagtgttttgttttgagaactgctatttatcttccattgtgcagGGTTACGCTGTG-
GAAGAAACAACGGGGATATTAGTAGCAGGAGACGTTGATGTGATGTCTATCACAGTGACATCCCTACCCTTAACA-
CATCCTAGCTACTACCCTGAGTTAGTTTTGGAATCGGGGGACATTTGGAAGGCACCACCTGTCCCAGCTAC-
CAAGATAGATTTATTTATTGGGATCATGTCCAGCAGTAACCATTTTGCAGAACGGATGGCAGTAAGGAAGACGTG-
GTTTCAATCTAAAGCTATTCAATCTTCGCAGGCCGTGGCTCGCTTCTTTGTAGCTCTGgtacttcctcctat-
caaatctcattaactttcgaattattagtgatcatctacataagtggtctgttgattgctgaaaggtggctgt-
tgcgtgcctttgcgtaatgactttccaaattcatttagaacagtggaaacataatttgtgtgttgcgt-
tgcgtatttaactttttcggtgaatgtcttattgaattgtgatgtagCATGCAAACAAGGATATCAATATGCAGT-
TGAAGAAGGAGGCAGACTATTATGGCGATATTATAATCCTGCCTTTCATCGACAGATATGATATAGTGGTTCT-
CAAGACCGTTGAAATTTGCAAGTTTGGGgtacgtgtgtcgaataatggcttcaaagctttgtgacggtgtct-
gcaatttggggatggtgataatgaggcttgataccaactgaaggttaggtgacttttaacactaggttctgct-

tactgtgcagGTCCAGAATGTCACAGCTAAGTATATTATGAAGTGTGACGATGACACTTTTGTGAGGAT-
TGATAGCGTTCTCGAAGAGATTCGAACTACTTCAATATCACAAGGCCTTTACATGGTAGCATGAAT-
GAGTTTCACAGGCCTCTTCGTTCTGGAAAGTGGGCCGTGACTGCCGAGgtatttttatttttattttg-
gcttttgtcgggaacgtgagagaaaccaagatgaatataatcacgatgttgtttttattgcaaggatttatttg-
atgctcttgagaaatctgtggtagccataccactcaatttggatactagatgtgttcgtccttatgtataaaaat-
gaaacatgtgcttttcaggaagattaattcagtttgacttgtacgtctagttagattgatggtgatgaaacaagag-
gattatctcgcgaattgacaagtgggttgcttggacagGAATGGCCTGAGCGAATTTACCCAATATATGCTAATG-
GACCAGGATATATCCTGTCAGAGGATATTGTGCATTTCATTGTGGAGATGAATGAGAGAGGCAGTTTGCAGgtag-
gttcttttagaactgtgtcgtcgctattacacgtctacaagttttaaaaattagaaactttcttgttggcaaatttccatccaggaatctttttgcaccgcaagttcgtaatag-
gagtcggtacattctgtgtgtgtgcatcgtttgttaaatgcatttttcaattttcttttgcttaaaatatctctgttgtcgatatctcctcatgatcttgcattgtgaacat-
gagaagatatgaaatgtgaactcaatattcttctatgatcatgtgcagTTATTTAAGATGGAGGACGTCAGTGTTGGAATATGG-
GTACGCGAATATGCGAAGCAAGTGAAGCACGTTCAATACGAACATAGCATACGGTTTGCTCAAGCCGGTTGTATAC-
CGAAATACTTGACAGCTCATTACCAATCGCCGCGTCAAATGCTGTGTCTGTGGGACAAGGTACTTGCTCATGACG-
ATGGGAAATGCTGCAACTTGTGAGGAAAATACATACAATGAATGTGTTCAACGGTCTTTACCAGACAGAAT-
TACTTTGGGTCGGGAACCAGATATAGCAGACAGCTCACATTCAATTCAGCCGTGTTGATCCAGAGGGGTAAT-
TGATAGTTTCCTTGTCCCCTACCCTCTCTAGAGGTGGAGATCTTACAACTTAATCAAATGATCCTCTGCAATGT-
CACTTGTCACAATACTTAGTATAGCTCAAAATTGGCCACGGATATTCAGGAATGTTCATCTTGTAAGGTCGCAGCT-
TGTGAGTAAATGGTTGGGTGGTGTCGATGGCATGGTTGCTTATCAATCCCTCTTAGCATCAGTGATCGTCAGAAT-
CAGTGTTTTCGACACTCCCCGGTGGAGTATTTTTTCGATTCTCTTGATTCCACTCAAGTGGTACTAGCTTATA-
TTTAGTGAGGCCTGGAACCCAAGTAGTTAGTTCAGTACGTCTGCCTTTTGCCGAAATGAGTAGAGTAATTTGTG-
GCAGTAGTTGGTGAAGAGACATGGTTAGGATTTAGTGTTCAAAATCTG 3´

SEQ ID NO: 4

Genomic DNA Ppβ1-3GalT2

ATGAAGAGGGGTGTGAGACCACCGGGTGTGGGATGTACAGGGCGGCAAAGAAACAATCTAATCATAGTGGCAAT-
CATATGTTTGGTTTTTATAGCGATATTCATCCCACCGTTTCTTGAAATGAATTCACTTCCCGATATTGATTCCCCT-
gtgtataggttagaaggtattaacttcgcttcacatagacgtcgctatcaagaacaggattcacgtgtcagt-
tacagtggctatggacagccagatatgccatcaactggtgatgaagacataacgaagacac-
cgtctaaagcttcacaggttagtgcagaaatgattggttcgccctcgctatgccagtcaggcttactgagttc-
tacttggatcgttctacttggatcttttatggcttcctagcagtcggaggtttctttctggtttgaagaaagccat-
gtatggaacgtttacagGTTTTGGAGAAGAAAGTATCAAGCTATTTGAAAAAAGTCACTCTGGAAACT-
TACAGTAAAGAGGAACGCCGTAGTCCAGGGAACACAACAGGTGACATTGTTTCGCTGGAAGATGTGATAG-
ATCGCGCCTGGTCTGCCGGCGCCAAAGCTTGGGAAGAGCTGGAAATTGCATTCAGACAGGGAGAA-
CATTTTTCGAAGAAGGACAATAATGCCAATGCAACTGCAGATCCATGCCCAGCATCACTCTTTACAACAGGAAAG-
GAATTGGACAATTTAGGAAGGGTCTTCCCACTGCCTTGTGGTCTAATGTTTGGATCAGCCATAACTCTCATTG-
GAAAGCCACGGGAAGCTCACATGGAGTACAAACCGCCAATCGCCAGAGTTGGGGAAGGTGTCTCTCCATACGTCAT-

GGTGTCCCAGTTCATAATGGAGTTACAGGGCTTGAAGGTGGTAAAGGTGAAGATCCTCCTAGAATCCTCCA-
CATAAACCCTCGACTCCGTGGTGACTGGAGCTGGAAACCCATCATTGAGCATAATACATGCTATCGAAACCAGT-
GGGGCCCAGCTCATCGGTGTGAAGGTTGGCAAGTACCTGAATACGAAGAAACCGgtgagtgctggttccat-
cacactttatcttttcatagtgacacggttcttttaggtgtactagtgttgaaagctgtgcatgttaaatg-
gtaaccctaatcaatcttctcgctaatttcgcattgcaaggtctccgctgcttggacaatcagcactctaacat-
tggctgtatttactgaaatgattctttactttgtagTGGACGGTCTTCCCAAGTGCGAGAAGTGGCTTCGAGGCG-
ATGACAAAAAACCTGCTTCGACCCAAAAATCCTGGTGGCTTGGGCGATTAGTTGGTCATTCCGACAAGGAGACGCT-
TGAATGGGAGTATCCATTGTCCGAAGGTCGGGAGTTTGTTCTCACCATTCGAGCAGGTGTAGAAGGATTTCACT-
TAACTATTGATGGTCGGCACATCAGTTCGTTCCCTTATCGTGCGgtgagttgaaaatactagtttgatatctaatg-
atgaggtttaccgcaggtatatttggtctcattgtcaagtgtgtgtgtgtgtgt-
tgtttttctttttcctttcatttctgaatcataatgataagaaatcaattctatgaaacttagcgtcaata-
ttttaaagttttattgtttttgtttgtttttatttttttgtgttttgtgttttgtgtttatttcacaatacaatgt-
taacaatggaatagaaacaatgatggtcccacctcacagacaccaggtacactacctacaccagactgcgtct-
gagtaagtttaagaaacagcaaccaccaacaatctgattgtaaattctaaattccttctccaccagaaaaccatgt-
gatccgtcttgcagttctgcttgcactctacctatatgatccaaagagtaattcctcttaacaggagttataacct-
gctggggttttgaaaataccgatgagttcaaattgtaaacaaaccccggatctatttcaagggtatgaagggct-
tagctttgtttaagaataaggtcaagagtatctgtgtggtgagcatcccaaaatggatgcaaatttgttaattg-
gcaactgttttctgtggtatgttttgtgacgcactatttattgtgtattgtgcagGGTTATGCTATG-
GAAGAAGCAACAGGAATATCAGTGGCAGGAGACGTCGATGTTCTTTCGATGACAGTAACATCATTACCTTTAACA-
CATCCCAGCTACTACCCTGAGTTGGTTTTGGATTCGGGTGATATCTGGAAGGCACCACCTTTACCAACAG-
GCAAGATAGAGTTATTTGTTGGAATCATGTCAAGCAGCAATCACTTTGCAGAACGTATGGCAGTAAGAAAGACGTG-
GTTTCAGTCTCTGGTTATCCAATCCTCCCAAGCGGTGGCTCGCTTCTTTGTAGCTCTGgtacttgtcat-
tatactctttttcgtgccaagtatcgtgaactcgggaatatttaaaaagtgcaaacaacaagtgagctgttaat-
tgctgaaaattggtgttataagtcttgatgcagtgaccttccagattgaccaagtatatcagacct-
tagaatttgaacagcactacttacttaccatttttaatgaatcccttgttgggttgtgatgcagCATGCAAACAAG-
GATATCAATCTGCAGCTGAAGAAAGAGGCTGACTATTACGGCGATATGATAATTTTACCTTTCATCGACAGATA-
TGATATAGTGGTTCTTAAGACCGTTGAAATTTTCAAGTTTGGGgtaagcgaat-
taaaatttgtagtatttacaaagtaatatttttaaacgttgtgaggacatctgcaacttgatatatttctttcgt-
gaggttcgatgctgattaaagcttaggtgatttaaaagcacggtgttgcttgctatgcagGTCCAGAATGT-
TACAGTTAGCCACGTCATGAAATGTGACGATGACACATTTGTAAGGATTGACAGCGTTCTTGAA-
GAGATTCGAACGACGTCAGTAGGACAGGGCCTTTACATGGGCAGCATGAATGAGTTTCATAGACCCCTTCGTTCT-
GGGAAGTGGGCCGTGACAGTTGAGgtaattttccctgtaccaaattatccaagattttcgtaaccattgtgtgcct-
tattcatttcttctgaaatctcaagaaaaatgaaaaatgcttgagaaacgctcgtagccgtatcacattat-
gcgaattccaaaaaagaatgtggaacaaaagttcttgtgaaaataattgatatgttcaaattgtacacatttat-
gcactaagataagatatgtgcaaatagtgccttccagtggtctagaaaatgcttgtttttttttg-
gaagctttaactttatttagcttgaacatcttgtttgagggttggtgaccaagtaagaag-
gtccatacaagacaataaatggattggttcgtgcatgtacagGAGTGGCCTGAGCGCATTTACCCAA-
CATACGCAAATGGTCCAGGATACATCCTTTCGGAAGATATTGTGCATTTTATAGTGGAGGA-
GAGCAAAAGAAATAATTTGAGGgtgcgtttttcatagctgtgtcctggtgattaaatgccccatgttcaacat-
tgaaaccttcatcttggacagttttccatccatgtatctcctgtcattataattgcattatagaactgttcgcgt-

gtacatttctttcctgttcctctttttcattttctttttctcttcttttcttcatttacttctcctcttgtcgat-
gctttctgttgaccttatattgtggatatgtatctcttcagtactacggagacgatatgaaacataagtttgata-
ttcttctgtgataaagcgcagTTATTTAAGATGGAGGACGTCAGCGTAGGTATATGGGTACGCGAGTATGCAAAG-
ATGAAGTACGTGCAATACGAGCATAGCGTACGGTTTGCTCAAGCCGGTTGTATACCTAACTACCT-
GACAGCGCACTATCAATCGCCGCGTCAAATGCTGTGTCTGTGGGACAAGGTGCTTGCTACCAATGACGGCAAGT-
GCTGCACCTTGTGA

## Claims

1. DNA molecules, **characterized in that** it comprises a sequence according to SEQ ID NO: 1 with an open reading frame from base pair 513 to base pair 2417 or a sequence according to SEQ ID NO: 2 with an open reading frame from base pair 1 to base pair 1902 or has at least 50% identity with at least one of the above sequences, or comprises a sequence which is degenerated to the above sequences due to the genetic code, with the sequences coding for plant proteins having β1,3-galactosyltransferase activity (β1,3-GalT activity) or being complementary thereto.

2. A DNA molecule according to claim 1, **characterized in that** it codes for a protein having GlcNAc-β1,3-galactosyltransferase activity.

3. A DNA molecule according to claim 1 or 3, **characterized in that** it codes for a protein having activity in respect to the transfer of galactose from UDP-galactose to non-reducing GlcNAc residues.

4. A DNA molecule according to any one of claims 1 to 3, **characterized in that** it codes for a protein having activity in respect to the transfer of galactose from UDP-galactose to non-reducing GlcNAc residues of N-glycan structures linked to proteins.

5. A DNA molecule according to claim any one of claims 1 to 4, **characterized in that** it has at least 70%, preferably at least 80%, especially at least 90%, identity with one of the sequences according to SEQ ID NO: 1 or SEQ ID NO: 2.

6. A DNA molecule, **characterized in that** it comprises a partial sequence of a DNA molecule according to any one of claims 1 to 5 and has at least 80% identity with the sequences according to SEQ ID NO: 1 or SEQ ID NO: 2 and has a size of 15 to 300, preferably 20 to 50 base pairs.

7. A DNA molecule according to any one of claims 1 to 6, **characterized in that** it is covalently associated with a detectable marker substance.

8. An expression vector, **characterized in that** it comprises a DNA molecule according to any one of claims 1 to 7.

9. An expression vector, **characterized in that** it comprises a DNA molecule according to any one of claims 1 to 8 being inversely oriented with respect to the promoter.

10. A DNA molecule coding for a ribozyme, **characterized in that** it has two sequence sections, each of which has a length of at least 10 to 15 base pairs and which are complementary to the sequence sections of a DNA molecule according to any one of claims 1 to 7 so that said ribozyme complexes and cuts the mRNA transcribed by a natural β1,3-GalT molecule.

11. A biologically functional vector, **characterized in that** it comprises a DNA molecule according to claim 10.

12. A method of cloning β1,3-galactosyltransferase, **characterized in that** a DNA molecule according to any one of claims 1 to 5 is cloned into a vector subsequently transfected into a host cell, a host tissue or a host with cell lines being obtained by means of selection and amplification of transfected host cells, which cell lines express the active β1,3galactosyltransferase.

13. A method of cloning β1,3-galactosyltransferase, **characterized in that** a DNA molecule according to any one of claims 1 to 5 but lacking at least the transmembrane encoding sequence is cloned into a vector subsequently transfected into a host cell, a host tissue or a host with cell lines being obtained by means of selection and amplification of transfected host cells, which cell lines express the active β1,3galactosyltransferase.

14. A protein according to claim 12 or 13, **characterized in that** it is active in respect to β1,3-galactosyltransferase and is used for elongation of N-glycans of glycoproteins *in vitro* or *in vivo*.

15. A transmembrane domain encoding DNA sequence according to any one of claims 1 to 5, **characterized in that** the encoded transmembrane domain is operably linked to a heterologous protein, particularly preferable an enzyme, more particularly preferable an enzyme involved in posttranslational modification of proteins.

16. DNA vector comprising a molecule with a nucleic acid sequence according to SEQ ID NO. 3 or SEQ ID No. 4.

17. A method of preparing recombinant host cells, particularly plant cells, or plants, respectively, wherein the production of β1,3-galactosyltransferase is suppressed, or completely stopped, respectively, **characterized in that** at least one of the vectors according to claims 8, 9, 11 or 16, or a vector comprising a DNA molecule according to any one of claims 1 to 7, respectively, whereby said DNA molecule comprises a deletion, insertion and/or substitution mutation, is inserted into said host cell, or plant, respectively. Ist die genomische Sequenz (claim 16) hier berücksichtigt?

18. A method of preparing recombinant host cells, particularly plant cells, or plants, respectively, **characterized in that** the DNA molecule according to any one of claims 1 to 7, whereby said DNA molecule comprises a deletion, insertion and/or substitution mutation, is inserted into the genome of said host cell, or plant, respectively, at the position of the non-mutated, homologous sequence.

19. Recombinant plants or plant cells, **characterized in that** at least one of the vectors according to claim 8, 9, 11 or 16, or a vector comprising a DNA molecule according to any one of claims 1 to 7, respectively, whereby said DNA molecule comprises a deletion, insertion and/or substitution mutation, is inserted thereinto, and that their endogenous β1,3-galactosyltransferase production is suppressed, or completely stopped, respectively.

20. Recombinant plants or plant cells, respectively, **characterized in that** the DNA molecule according to any one of claims 1 to 7, whereby said DNA molecule comprises a deletion, insertion and/or substitution mutation, is inserted into the genome of the cells or plants, respectively, at the site of the non-mutated, homologous sequence, and **in that** their endogenous β1,3-galactosyltransferase production is suppressed, or completely stopped, respectively.

21. A peptide nucleic acid (PNA) molecule, **characterized in that** it comprises a base sequence complementary to the sequence of a DNA molecule according to any one of claims 1 to 6 which codes for β1,3-galactosyltransferase.

22. A peptide nucleic acid (PNA) molecule, **characterized in that** it comprises a base sequence corresponding to the sequence of a DNA molecule according to any one of claims 1 to 6 which codes for β1,3-galactosyltransferase.

23. A method of producing plants, or cells, respectively, particularly plant cells having blocked expression of β1,3-galactosyltransferase at the transcription or translation level, respectively, **characterized in that** PNA molecules according to claim 21 or 22 are inserted into the cells.

24. A method of producing recombinant glycoproteins, **characterized in that** the recombinant plants, plant cells or plant tissues, respectively, according to claim 19 or 20, or plants, plant tissues or cells, respectively, in which the PNA molecule according to claim 21 or 22 is inserted and which have a blocked expression of the β1,3-galactosyltransferase at the transcription or translation level, respectively, are transfected with the gene that codes for the glycoprotein, so that the recombinant glycoproteins are expressed.

25. A method of producing recombinant human glycoproteins, **characterized in that** the recombinant plants, plant cells or plant tissues, respectively, according to claim 19 or 20 or plants, plant tissues or cells, respectively, in which the PNA molecule according to claim 21 or 22 is inserted and which have a blocked expression of the β1,3-galactosyltransferase at the transcription or translation level, respectively, are transfected with the gene that codes for the glycoprotein, so that the recombinant glycoproteins are expressed.

26. A method of producing recombinant human glycoproteins for medical use, **characterized in that** the recombinant plants, plant cells or plant tissues, respectively, according to claim 19 or 20 or plants, plant tissues or cells, respectively, in which the PNA molecule according to claim 21 or 22 is inserted and which have a blocked expression of the β1,3-galactosyltransferase at the transcription or translation level, are transfected with the gene that codes for the glycoprotein, so that the recombinant glycoproteins are expressed.

27. A method of selecting DNA molecules coding for a β1,3-galactosyltransferase, in a sample, **characterized in that** DNA molecules according to claim 7 are added to said sample, which molecules bind to the DNA molecules coding for a β1,3-galactosyltransferase.

28. A method according to claim 27, **characterized in that** said sample comprises genomic DNA of a plant organism.


SEQUENCE LISTING

<110>  Greenovation Biotech GmbH

<120>  Galactosyltransferase

<130>  r48673

<140>  EP 06450139.8
<141>  2006-09-29

<160>  23

<170>  PatentIn version 3.3

<210>  1
<211>  2957
<212>  DNA
<213>  Physcomitrella patens

<220>
<221>  misc_feature
<222>  (432)..(432)
<223>  n is a, c, g, or t

<400>  1
agttgtcgat ttgttgtttt tgatatgtaa ggcggttgcc ttcgcgccgt gcttgattgt      60

aattgtaatt caatctggag tgtgagatat atatatatat atatatatag cgagagggag     120

agagaaagag agagagaggg agagagaaag agagagagag ggagagagag agatggcttg     180

tgtatgaggg ccatgcgagg aggaggctgt gtttgttgcc cgaagagatg ggatggttta     240

tgtgtagtgc aggggttgga tgtgaagcac ctgtttgaag gagtctgcga gagtttgaaa     300

ttcggattca gagtgcggcg atcgatggtg caacgttgtt agcagtgatt gttttcgcca     360

acagaactga catcatttgg atttttttta cgcgtggatg tgccctcttt ttaaaaaatt     420

tccgcgtgga anagagacgg gggtttgtaa tggaggcagg ctgtggtcat cacccctagt     480

atagcctgtc aagagagttc aaattcggta atatgaagag ggggtcgaga ctaccggata     540

tggcgtgtac agggcggcaa agaaatgatc ttatcctagt tgcaattgtt tgcttgtttt     600

ttatggtgat attcatccca ccatatctcc aaatgaactc acttccggac attgattctc     660

ctgattcgga caagaaatca tcaagctact cgaaaaaaac cactctagaa gccaatagta     720

aggaggaacg ccgtagtccg gggaatacca caggcgacat tgtttctctg gatgatgtga     780

tagatcgtgc ctggtctgct ggtgccaaag cgtgggaaga actggaaact gcgttaagaa     840

atggagaagg tgtctcaaag aatgtcagta atgccactgc aaatgctgat ccgtgtccag     900

catcactctc tgcagcaggg aaaaagttag acgaattggg taaagtcttc cccttgccct     960

gtggtctaat gtttgggtca gccattactc tgattggaaa gcctcgagag gctcacatgg    1020

agtacaaacc gccaatcgcc agagttgggg aaggcgtctc tccatatgtc atggtttccc    1080

agttcttagt agagttacaa ggcttaaagg tggtgaaagg tgaagatcct cctcgaattc    1140

tacacttgaa tcctcgactt cgtggtgatt ggagctggaa acccatcatt gagcacaaca    1200

cttgttatcg gaaccagtgg ggtcctgccc accgatgcga gggttggcaa gtgcctgaat    1260

acgaagaaac tgttgacggt cttcccaagt gcgagaagtg gcttcgagat gatggcaaga    1320

aacctgcttc aacgcaaaaa tcttggtggc ttggaagatt agttggtcgt tctgacaagg 1380

agacgcttga atgggagtac ccattatctg agggtcggga gttcgttctc accattcgag 1440

caggtgttga agggtttcat gtgactatcg atggtcgtca catcagctcg tttccttatc 1500

gtgtgggtta cgctgtggaa gaaacaacgg ggatattagt agcaggagac gttgatgtga 1560

tgtctatcac agtgacatcc ctacccttaa cacatcctag ctactaccct gagttagttt 1620

tggaatcggg ggacatttgg aaggcaccac ctgtcccagc taccaagata gatttattta 1680

ttgggatcat gtccagcagt aaccattttg cagaacggat ggcagtaagg aagacgtggt 1740

ttcaatctaa agctattcaa tcttcgcagg ccgtggctcg cttctttgta gctctgcatg 1800

caaacaagga tatcaatatg cagttgaaga aggaggcaga ctattatggc gatattataa 1860

tcctgccttt catcgacaga tatgatatag tggttctcaa gaccgttgaa atttgcaagt 1920

ttggggtcca gaatgtcaca gctaagtata ttatgaagtg tgacgatgac acttttgtga 1980

ggattgatag cgttctcgaa gagattcgaa ctacttcaat atcacaaggc ctttacatgg 2040

gtagcatgaa tgagtttcac aggcctcttc gttctggaaa gtgggccgtg actgccgagg 2100

aatggcctga gcgaatttac ccaatatatg ctaatggacc aggatatatc ctgtcagagg 2160

atattgtgca tttcattgtg gagatgaatg agagaggcag tttgcagtta tttaagatgg 2220

aggacgtcag tgttggaata tgggtacgcg aatatgcgaa gcaagtgaag cacgttcaat 2280

acgaacatag catacggttt gctcaagccg gttgtatacc gaaatacttg acagctcatt 2340

accaatcgcc gcgtcaaatg ctgtgtctgt gggacaaggt acttgctcat gacgatggga 2400

aatgctgcaa cttgtgagga aaatacatac aatgaatgtg ttcaacggtc tttaccagac 2460

agaattactt tgggtcggga accagatata gcagacagct cacattcaat tcagccgtgt 2520

tgatccagag gggtaattga tagtttcctt gtcccctacc ctctctagag gtggagatct 2580

tacaacttaa tcaaatgatc ctctgcaatg tcacttgtca caatacttag tatagctcaa 2640

aattggccac ggatattcag gaatgttcat cttgtaaggt cgcagcttgt gagtaaatgg 2700

ttgggtggtg tcgatggcat ggttgcttat caatccctct tagcatcagt gatcgtcaga 2760

atcagtgttt tcgacactcc ccggtggagt attttttcga ttctcttgat tccactcaag 2820

tggtactagc ttatatttag tgaggcctgg aacccaagta gttagttcag tacgtctgcc 2880

ttttgccgaa atgagtagag taatttgtgg cagtagttgg tgaagagaca tggttaggat 2940

ttagtgttca aaatctg 2957

<210> 2
<211> 1902
<212> DNA
<213> Physcomitrella patens

<400> 2
atgaagaggg gtgtgagacc accgggtgtg ggatgtacag ggcggcaaag aaacaatcta 60

atcatagtgg caatcatatg tttggttttt atagcgatat tcatcccacc gtttcttgaa 120

atgaattcac ttcccgatat tgattcccct gttttggaga agaaagtatc aagctatttg 180

aaaaaagtca ctctggaaac ttacagtaaa gaggaacgcc gtagtccagg gaacacaaca 240
ggtgacattg tttcgctgga agatgtgata gatcgcgcct ggtctgccgg cgccaaagct 300
tgggaagagc tggaaattgc attcagacag ggagaacatt tttcgaagaa ggacaataat 360
gccaatgcaa ctgcagatcc atgcccagca tcactcttta caacaggaaa ggaattggac 420
aatttaggaa gggtcttccc actgccttgt ggtctaatgt ttggatcagc cataactctc 480
attggaaagc cacgggaagc tcacatggag tacaaaccgc caatcgccag agttggggaa 540
ggtgtctctc catacgtcat ggtgtcccag ttcataatgg agttacaggg cttgaaggtg 600
gtaaaaggtg aagatcctcc tagaatcctc cacataaacc ctcgactccg tggtgactgg 660
agctggaaac ccatcattga gcataataca tgctatcgaa accagtgggg cccagctcat 720
cggtgtgaag gttggcaagt acctgaatac gaagaaaccg tggacggtct tcccaagtgc 780
gagaagtggc ttcgaggcga tgacaaaaaa cctgcttcga cccaaaaatc ctggtggctt 840
gggcgattag ttggtcattc cgacaaggag acgcttgaat gggagtatcc attgtccgaa 900
ggtcgggagt ttgttctcac cattcgagca ggtgtagaag gatttcactt aactattgat 960
ggtcggcaca tcagttcgtt cccttatcgt gcgggttatg ctatggaaga agcaacagga 1020
atatcagtgg caggagacgt cgatgttctt tcgatgacag taacatcatt acctttaaca 1080
catcccagct actaccctga gttggttttg gattcgggtg atatctggaa ggcaccacct 1140
ttaccaacag gcaagataga gttatttgtt ggaatcatgt caagcagcaa tcactttgca 1200
gaacgtatgg cagtaagaaa gacgtggttt cagtctctgg ttatccaatc ctcccaagcg 1260
gtggctcgct tctttgtagc tctgcatgca aacaaggata tcaatctgca gctgaagaaa 1320
gaggctgact attacggcga tatgataatt ttacctttca tcgacagata tgatatagtg 1380
gttcttaaga ccgttgaaat tttcaagttt ggggtccaga atgttacagt tagccacgtc 1440
atgaaatgtg acgatgacac atttgtaagg attgacagcg ttcttgaaga gattcgaacg 1500
acgtcagtag gacagggcct ttacatgggc agcatgaatg agtttcatag accccttcgt 1560
tctgggaagt gggccgtgac agttgaggag tggcctgagc gcatttaccc aacatacgca 1620
aatggtccag gatacatcct ttcggaagat attgtgcatt ttatagtgga ggagagcaaa 1680
agaaataatt tgaggttatt taagatggag gacgtcagcg taggtatatg ggtacgcgag 1740
tatgcaaaga tgaagtacgt gcaatacgag catagcgtac ggtttgctca agccggttgt 1800
atacctaact acctgacagc gcactatcaa tcgccgcgtc aaatgctgtg tctgtgggac 1860
aaggtgcttg ctaccaatga cggcaagtgc tgcaccttgt ga 1902

<210> 3
<211> 6187
<212> DNA
<213> Physcomitrella patens

<400> 3
agttgtcgat ttgttgtttt tgatatgtaa ggcggttgcc ttcgcgccgt gcttgattgt 60
aattgtaatt caatctggag tgtgagatat atatatatat atatatatag cgagagggag 120
agagaaagag agagagaggg agagagaaag agagagagag ggagagagag agatggcttg 180

tgtatgaggg ccatgcgagg aggaggctgt gtttgttgcc cgaagagatg ggatggttta 240
tgtgtagtgc aggggttgga tgtgaagcac ctgtttgaag gagtctgcga gagtttgaaa 300
ttcggattca gagtgcggcg atcgatggtg caacgttgtt agcagtgatt gttttcgcca 360
acagaactga catgtaatga atagtttcga ggcatgatcg cggtttttct caatttgaag 420
gggttgtttg tgggtgatct atgtgcagaa gtgtcactga tggtcagatt cgatgcttga 480
caatttgatc ctttgtgagt gtgcagcatt tggatttttt ttacgcgtgg atgtgccctc 540
tttttaaaaa atttccgcgt ggaaaagaga cgggggtttg taatggaggc aggctgtggt 600
catcacccct agtatagcct gtcaagaggt gagattgaca ccctctttgc tcaattgtag 660
atttttttcc ttctcagggc tgaatcccag tttttttttt tttttttttt tttttttcct 720
tcttcttcaa cttcgagttc gtgtctgtat gaagaagtcc acgggttcaa tgtgttaaga 780
cttaggcatt tccttcagct ttgcctagtg gagatatgcg tattttttga ttgtgaggat 840
tccggttctt agaccatgat tggtttatta cagtggtcat tcaaatccta tttgatttga 900
gaatgtattt acttcgttgt gttgggagat gattgttccc tcgaattcta tgcggtagct 960
accgcttctt tcgtaatgaa gacctttgaa gttcacatag acttcaagaa gaatgctatt 1020
tgtgtttttg tgattgtgtg ttcaagtttg gtgcagtatt gttaaaattt gggtgatgac 1080
taagtacact ttatgcggcc caagtagtca agttgagcat ttgtaaatgc tgaaatgagt 1140
taggctgacg gtaaatgtct gtggatgtag cctagtgatg tatttgatct cggcataatc 1200
ttcagtgatc aatacaaata attcaagaaa gaggggtcaa tgtgttcctg cgagtacctt 1260
cgcatgttca acgtgaactg aattatgtta attaagctga gcaacataga ccttcttgct 1320
gttgacagag ttcaaattcg gtaatatgaa gagggggtcg agactaccgg atatggcgtg 1380
tacagggcgg caaagaaatg atcttatcct agttgcaatt gtttgcttgt tttttatggt 1440
gatattcatc ccaccatatc tccaaatgaa ctcacttccg gacattgatt ctcctgtcga 1500
gaagctagaa gatgatgatg atgctgtctt cacttctcat agacgtcgta accaagagca 1560
gatttcagtt gtcactgaca gtggtcagag acggacagtt atgccatctt cgactggtgc 1620
ggaggacgta acgaatgcac cgtctaaaga ttcacaggtt agaccaaaag tagttgacct 1680
gaaatgcatg tggtaatcaa gcactcttgt ccttattcga gcttttattt cttgccatca 1740
ggtattttta atacttccct agtgtacgct gagtgtctac attgtgtatt gaatgttcct 1800
tagaattgtt tgtttgttta tgtttttatt tttatatttc tgccggctat tgaggaagaa 1860
tacattcaaa ttgttcagga ttcggacaag aaatcatcaa gctactcgaa aaaaaccact 1920
ctagaagcca atagtaagga ggaacgccgt agtccgggga ataccacagg cgacattgtt 1980
tctctggatg atgtgataga tcgtgcctgg tctgctggtg ccaaagcgtg ggaagaactg 2040
gaaactgcgt taagaaatgg agaaggtgtc tcaaagaatg tcagtaatgc cactgcaaat 2100
gctgatccgt gtccagcatc actctctgca gcagggaaaa agttagacga attgggtaaa 2160
gtcttcccct tgccctgtgg tctaatgttt gggtcagcca ttactctgat tggaaagcct 2220
cgagaggctc acatggagta caaaccgcca atcgccagag ttggggaagg cgtctctcca 2280

tatgtcatgg tttcccagtt cttagtagag ttacaaggct taaaggtggt gaaaggtgaa 2340
gatcctcctc gaattctaca cttgaatcct cgacttcgtg gtgattggag ctggaaaccc 2400
atcattgagc acaacacttg ttatcggaac cagtggggtc ctgcccaccg atgcgagggt 2460
tggcaagtgc ctgaatacga agaaactggt gagtgctgat tccaccgcac cagtttgtgt 2520
tttttatgct gacactatgc ttctcaggtt tgtagacgtt aagagctgtg taggttccgt 2580
ggtacttcga attggcactt gccacttctc tcattgtaag ttggtaaatg tctgcatgag 2640
caataaattc caacactgga tgtgtatttt ctgaaatgat tcgttttctt gtagttgacg 2700
gtcttcccaa gtgcgagaag tggcttcgag atgatggcaa gaaacctgct tcaacgcaaa 2760
aatcttggtg gcttggaaga ttagttggtc gttctgacaa ggagacgctt gaatgggagt 2820
acccattatc tgagggtcgg gagttcgttc tcaccattcg agcaggtgtt gaagggtttc 2880
atgtgactat cgatggtcgt cacatcagct cgtttcctta tcgtgtggta agttgaaaat 2940
gctatgttaa catataatgc taaagttgac ctcatgtctt tctttttctt ttttttcttt 3000
tttattttct ggaggggggg ggggtaatgc aaatcaactc taaaatttta gtataccagt 3060
taaattattc atttcaaata taacaataca aatacacatc tttttaattt gtattttttg 3120
atccctctcc tcctctacta aaattaataa tatagcaaca ttttggtact acgaaagttc 3180
atttgtattg cttcatgtcg aagatttatt caaaatttct atccctcgtg tttctgaatt 3240
acattatcaa caatggaata acaataatga cggccccatc cttcagacac caggaacatt 3300
acctatacca gactacgtct gggtaagtct gaagaattaa ttataaccaa gaaactagtt 3360
gtattcactg tttttctttt tacgcccatg cgatttatcg aagtcttctt caatttctta 3420
ttattcttct ttattatttt aagtttttaa ttatttttaa agcaacgaat tgataaataa 3480
ataacatatt aatgttttta actttaaagt ttttttcccg tatttagtat aagatttcgt 3540
caaaacgatt aggtgattag atcgaacatt atctaattgc actctactta tatgatatga 3600
agagtaattt ctcttagcag aagctacatc ctgctatttc cttgggaaac ccgattaggt 3660
ctttcaaatc acccctgctt cctctataag tgtaccatga ttgaggttcg ttagggcatt 3720
agtttaaggg tatcgttgtg atgtgtgtct agttagtctt aaaatctgtg caaatcgatt 3780
cattaacaac tcttttctgt agtgttttgt tttgagaact gctatttatc ttccattgtg 3840
cagggttacg ctgtggaaga aacaacgggg atattagtag caggagacgt tgatgtgatg 3900
tctatcacag tgacatccct acccttaaca catcctagct actaccctga gttagttttg 3960
gaatcggggg acatttggaa ggcaccacct gtcccagcta ccaagataga tttatttatt 4020
gggatcatgt ccagcagtaa ccattttgca gaacggatgg cagtaaggaa gacgtggttt 4080
caatctaaag ctattcaatc ttcgcaggcc gtggctcgct tctttgtagc tctggtactt 4140
cctcctatca aatctcatta actttcgaat tattagtgat catctacata agtggtctgt 4200
tgattgctga aaggtggctg ttgcgtgcct ttgcgtaatg actttccaaa ttcatttaga 4260
acagtggaaa cataatttgt gtgttgcgtt gcgtatttaa ctttttcggt gaatgtctta 4320
ttgaattgtg atgtagcatg caaacaagga tatcaatatg cagttgaaga aggaggcaga 4380

ctattatggc gatattataa tcctgccttt catcgacaga tatgatatag tggttctcaa 4440
gaccgttgaa atttgcaagt ttggggtacg tgtgtcgaat aatggcttca aagctttgtg 4500
acggtgtctg caatttgggg atggtgataa tgaggcttga taccaactga aggttaggtg 4560
acttttaaca ctaggttctg cttactgtgc aggtccagaa tgtcacagct aagtatatta 4620
tgaagtgtga cgatgacact tttgtgagga ttgatagcgt tctcgaagag attcgaacta 4680
cttcaatatc acaaggcctt tacatgggta gcatgaatga gtttcacagg cctcttcgtt 4740
ctggaaagtg ggccgtgact gccgaggtat tttatttttt atttttggct tttgtcggga 4800
acgtgagaga aaccaagatg aatataatca cgatgttgtt ttttattgca aggatttatt 4860
tgatgctctt gagaaatctg tggtagccat accactcaat ttggatacta gatgtgttcg 4920
tccttatgta taaaaatgaa acatgtgctt ttcaggaaga ttaattcagt ttgacttgta 4980
cgtctagtta gattgatggt gatgaaacaa gaggattatc tcgcgaattg acaagtgggt 5040
tgcttggaca ggaatggcct gagcgaattt acccaatata tgctaatgga ccaggatata 5100
tcctgtcaga ggatattgtg catttcattg tggagatgaa tgagagaggc agtttgcagg 5160
taggttcttt tagaactgtg tcgtcgctat tacacgtcta caagttttaa aaattagaaa 5220
ctttcttgtt ggcaaatttc catccaggaa tctttttgca ccgcaagttc gtaataggag 5280
tcggtacatt ctgtgtgtgt gcatcgtttg ttaaatgcat ttttcaattt tcttttgctt 5340
aaaatatctc tgttgtcgat atctcctcat gatcttgcat tgtgaacatg agaagatatg 5400
aaatgtgaac tcaatattct tctatgatca tgtgcagtta tttaagatgg aggacgtcag 5460
tgttggaata tgggtacgcg aatatgcgaa gcaagtgaag cacgttcaat acgaacatag 5520
catacggttt gctcaagccg gttgtatacc gaaatacttg acagctcatt accaatcgcc 5580
gcgtcaaatg ctgtgtctgt gggacaaggt acttgctcat gacgatggga aatgctgcaa 5640
cttgtgagga aaatacatac aatgaatgtg ttcaacggtc tttaccagac agaattactt 5700
tgggtcggga accagatata gcagacagct cacattcaat tcagccgtgt tgatccagag 5760
gggtaattga tagtttcctt gtcccctacc ctctctagag gtggagatct tacaacttaa 5820
tcaaatgatc ctctgcaatg tcacttgtca caatacttag tatagctcaa aattggccac 5880
ggatattcag gaatgttcat cttgtaaggt cgcagcttgt gagtaaatgg ttgggtggtg 5940
tcgatggcat ggttgcttat caatccctct tagcatcagt gatcgtcaga atcagtgttt 6000
tcgacactcc ccggtggagt attttttcga ttctcttgat tccactcaag tggtactagc 6060
ttatatttag tgaggcctgg aacccaagta gttagttcag tacgtctgcc ttttgccgaa 6120
atgagtagag taatttgtgg cagtagttgg tgaagagaca tggttaggat ttagtgttca 6180
aaatctg 6187

<210> 4
<211> 4087
<212> DNA
<213> Physcomitrella patens

<400> 4

atgaagaggg gtgtgagacc accgggtgtg ggatgtacag ggcggcaaag aaacaatcta 60
atcatagtgg caatcatatg tttggttttt atagcgatat tcatcccacc gtttcttgaa 120
atgaattcac ttcccgatat tgattcccct gtgtataggt tagaaggtat taacttcgct 180
tcacatagac gtcgctatca agaacaggat tcacgtgtca gttacagtgg ctatggacag 240
ccagatatgc catcaactgg tgatgaagac ataacgaaga caccgtctaa agcttcacag 300
gttagtgcag aaatgattgg ttcgccctcg ctatgccagt caggcttact gagttctact 360
tggatcgttc tacttggatc ttttatggct tcctagcagt cggaggtttc tttctggttt 420
gaagaaagcc atgtatggaa cgtttacagg ttttggagaa gaaagtatca agctatttga 480
aaaaagtcac tctggaaact tacagtaaag aggaacgccg tagtccaggg aacacaacag 540
gtgacattgt ttcgctggaa gatgtgatag atcgcgcctg gtctgccggc gccaaagctt 600
gggaagagct ggaaattgca ttcagacagg gagaacattt ttcgaagaag gacaataatg 660
ccaatgcaac tgcagatcca tgcccagcat cactctttac aacaggaaag gaattggaca 720
atttaggaag ggtcttccca ctgccttgtg gtctaatgtt tggatcagcc ataactctca 780
ttggaaagcc acgggaagct cacatggagt acaaaccgcc aatcgccaga gttggggaag 840
gtgtctctcc atacgtcatg gtgtcccagt tcataatgga gttacagggc ttgaaggtgg 900
taaaaggtga agatcctcct agaatcctcc acataaaccc tcgactccgt ggtgactgga 960
gctggaaacc catcattgag cataatacat gctatcgaaa ccagtggggc ccagctcatc 1020
ggtgtgaagg ttggcaagta cctgaatacg aagaaaccgg tgagtgctgg ttccatcaca 1080
ctttatcttt tcatagtgac acggttcttt ttaggtgtac tagtgttgaa agctgtgcat 1140
gttaaatggt aaccctaatc aatcttctcg ctaattttcg cattgcaagg tctccgctgc 1200
ttggacaatc agcactctaa cattggctgt atttactgaa atgattcttt actttgtagt 1260
ggacggtctt cccaagtgcg agaagtggct tcgaggcgat gacaaaaaac ctgcttcgac 1320
ccaaaaatcc tggtggcttg ggcgattagt tggtcattcc gacaaggaga cgcttgaatg 1380
ggagtatcca ttgtccgaag gtcgggagtt tgttctcacc attcgagcag gtgtagaagg 1440
atttcactta actattgatg gtcggcacat cagttcgttc ccttatcgtg cggtgagttg 1500
aaaatactag tttgatatct aatgatgagg tttaccgcag gtatatttgg tctcattgtc 1560
aagtgtgtgt gtgtgtgttg tttttctttt ttccttttca ttttctgaat cataatgata 1620
agaaatcaat tctatgaaac ttagcgtcaa tattttaaag ttttattgtt tttgtttgtt 1680
tttatttttt tgtgttttgt gttttgtgtt tatttcacaa tacaatgtta acaatggaat 1740
agaaacaatg atggtcccac ctcacagaca ccaggtacac tacctacacc agactgcgtc 1800
tgagtaagtt taagaaacag caaccaccaa caatctgatt gtaaattcta aattccttct 1860
ccaccagaaa accatgtgat ccgtcttgca gttctgcttg cactctacct atatgatcca 1920
aagagtaatt cctcttaaca ggagttataa cctgctgggg ttttgaaaat accgatgagt 1980
tcaaattgta aacaaacccc ggatctattt caagggtatg aagggcttag ctttgtttaa 2040
gaataaggtc aagagtatct gtgtggtgag catcccaaaa tggatgcaaa tttgttaatt 2100

ggcaactgtt ttctgtggta tgttttgtga cgcactattt attgtgtatt gtgcagggtt 2160
atgctatgga agaagcaaca ggaatatcag tggcaggaga cgtcgatgtt ctttcgatga 2220
cagtaacatc attaccttta acacatccca gctactaccc tgagttggtt ttggattcgg 2280
gtgatatctg gaaggcacca cctttaccaa caggcaagat agagttattt gttggaatca 2340
tgtcaagcag caatcacttt gcagaacgta tggcagtaag aaagacgtgg tttcagtctc 2400
tggttatcca atcctcccaa gcggtggctc gcttctttgt agctctggta cttgtcatta 2460
tactcttttt tcgtgccaag tatcgtgaac tcgggaatat ttaaaaagtg caaacaacaa 2520
gtgagctgtt aattgctgaa aattggtgtt ataagtcttg atgcagtgac cttccagatt 2580
gaccaagtat atcagacctt agaatttgaa cagcactact tacttaccat ttttaatgaa 2640
tcccttgttg ggttgtgatg cagcatgcaa acaaggatat caatctgcag ctgaagaaag 2700
aggctgacta ttacggcgat atgataattt tacctttcat cgacagatat gatatagtgg 2760
ttcttaagac cgttgaaatt ttcaagtttg gggtaagcga attaaaattt gtagtattta 2820
caaagtaata tttttaaacg ttgtgaggac atctgcaact tgatatattt ctttcgtgag 2880
gttcgatgct gattaaagct taggtgattt aaaagcacgg tgttgcttgc tatgcaggtc 2940
cagaatgtta cagttagcca cgtcatgaaa tgtgacgatg acacatttgt aaggattgac 3000
agcgttcttg aagagattcg aacgacgtca gtaggacagg gcctttacat gggcagcatg 3060
aatgagtttc atagacccct tcgttctggg aagtgggccg tgacagttga ggtaattttc 3120
cctgtaccaa attatccaag attttcgtaa ccattgtgtg ccttattcat ttcttctgaa 3180
atctcaagaa aaatgaaaaa tgcttgagaa acgctcgtag ccgtatcaca ttatgcgaat 3240
tccaaaaaag aatgtggaac aaaagttctt gtgaaaataa ttgatatgtt caaattgtac 3300
acatttatgc actaagataa gatatgtgca aatagtgcct tccagtggtc tagaaaatgc 3360
ttgttttttt ttggaagctt taactttatt tagcttgaac atcttgtttg agggttggtg 3420
accaagtaag aaggtccata caagacaata aatggattgg ttcgtgcatg tacaggagtg 3480
gcctgagcgc atttacccaa catacgcaaa tggtccagga tacatccttt cggaagatat 3540
tgtgcatttt atagtggagg agagcaaaag aaataatttg agggtgcgtt tttcatagct 3600
gtgtcctggt gattaaatgc cccatgttca acattgaaac cttcatcttg gacagttttc 3660
catccatgta tctcctgtca ttataattgc attatagaac tgttcgcgtg tacatttctt 3720
tcctgttcct ctttttcatt ttctttttct cttcttttct tcatttactt ctcctcttgt 3780
cgatgctttc tgttgacctt atattgtgga tatgtatctc ttcagtacta cggagacgat 3840
atgaaacata agtttgatat tcttctgtga taaagcgcag ttatttaaga tggaggacgt 3900
cagcgtaggt atatgggtac gcgagtatgc aaagatgaag tacgtgcaat acgagcatag 3960
cgtacggttt gctcaagccg gttgtatacc taactacctg acagcgcact atcaatcgcc 4020
gcgtcaaatg ctgtgtctgt gggacaaggt gcttgctacc aatgacggca agtgctgcac 4080
cttgtga 4087

<210> 5

<211>  18
<212>  DNA
<213>  Artificial

<220>
<223>  primer

<400>  5
ctgaatatcc gtggccaa                                                    18


<210>  6
<211>  29
<212>  DNA
<213>  Artificial

<220>
<223>  primer

<400>  6
ttcgagctca tgaagagggg gtcgagact                                        29


<210>  7
<211>  29
<212>  DNA
<213>  Artificial

<220>
<223>  primer

<400>  7
tacgagctca tgaagagggg tgtgagacc                                        29


<210>  8
<211>  28
<212>  DNA
<213>  Artificial

<220>
<223>  primer

<400>  8
gtagagctct cacaaggtgc agcacttg                                         28


<210>  9
<211>  30
<212>  DNA
<213>  Artificial

<220>
<223>  primer

<400>  9
tacggatcca acttcgagtt cgtgtctgta                                       30


<210>  10
<211>  33
<212>  DNA
<213>  Artificial

<220>
<223>  primer

<400>  10
acactaagct tctaatcaat gtccggaagt gag                                   33

<210> 11
<211> 34
<212> DNA
<213> Artificial

<220>
<223> primer

<400> 11
ttagaagctt agtgtacgct gagtgtctac attg 34

<210> 12
<211> 33
<212> DNA
<213> Artificial

<220>
<223> primer

<400> 12
cattgtcgac cctacacagc tcttaacgtc tac 33

<210> 13
<211> 1585
<212> DNA
<213> Artificial

<220>
<223> GalT knock-out

<400> 13
caacttcgag ttcgtgtctg tatgaagaag tccacgggtt caatgtgtta agacttaggc 60
atttccttca gctttgccta gtggagatat gcgtattttt tgattgtgag gattccggtt 120
cttagaccat gattggttta ttacagtggt cattcaaatc ctatttgatt tgagaatgta 180
tttacttcgt tgtgttggga gatgattgtt ccctcgaatt ctatgcggta gctaccgctt 240
ctttcgtaat gaagaccttt gaagttcaca tagacttcaa gaagaatgct atttgtgttt 300
ttgtgattgt gtgttcaagt ttggtgcagt attgttaaaa tttgggtgat gactaagtac 360
actttatgcg gcccaagtag tcaagttgag catttgtaaa tgctgaaatg agttaggctg 420
acggtaaatg tctgtggatg tagcctagtg atgtatttga tctcggcata atcttcagtg 480
atcaatacaa ataattcaag aaagaggggt caatgtgttc ctgcgagtac cttcgcatgt 540
tcaacgtgaa ctgaattatg ttaattaagc tgagcaacat agaccttctt gctgttgaca 600
gagttcaaat tcggtaatat gaagaggggg tcgagactac cggatatggc gtgtacaggg 660
cggcaaagaa atgatcttat cctagttgca attgtttgct tgttttttat ggtgatattc 720
atcccaccat atctccaaat gaactcactt ccggacattg attagaagct tagtgtacgc 780
tgagtgtcta cattgtgtat tgaatgttcc ttagaattgt ttgtttgttt atgtttttat 840
ttttatattt ctgccggcta ttgaggaaga atacattcaa attgttcagg attcggacaa 900
gaaatcatca agctactcga aaaaaaccac tctagaagcc aatagtaagg aggaacgccg 960
tagtccgggg aataccacag gcgacattgt ttctctggat gatgtgatag atcgtgcctg 1020
gtctgctggt gccaaagcgt gggaagaact ggaaactgcg ttaagaaatg gagaaggtgt 1080

ctcaaagaat gtcagtaatg ccactgcaaa tgctgatccg tgtccagcat cactctctgc 1140

agcagggaaa aagttagacg aattgggtaa agtcttcccc ttgccctgtg gtctaatgtt 1200

tgggtcagcc attactctga ttggaaagcc tcgagaggct cacatggagt acaaaccgcc 1260

aatcgccaga gttggggaag gcgtctctcc atatgtcatg gtttcccagt tcttagtaga 1320

gttacaaggc ttaaaggtgg tgaaaggtga agatcctcct cgaattctac acttgaatcc 1380

tcgacttcgt ggtgattgga gctggaaacc catcattgag cacaacactt gttatcggaa 1440

ccagtggggt cctgcccacc gatgcgaggg ttggcaagtg cctgaatacg aagaaactgg 1500

tgagtgctga ttccaccgca ccagtttgtg ttttttatgc tgacactatg cttctcaggt 1560

ttgtagacgt taagagctgt gtagg 1585

<210> 14
<211> 21
<212> DNA
<213> Artificial

<220>
<223> primer

<400> 14
tggcacgata cagtggcatg a 21

<210> 15
<211> 29
<212> DNA
<213> Artificial

<220>
<223> primer

<400> 15
tggaattcat tcaagaaacg gtgggatga 29

<210> 16
<211> 27
<212> DNA
<213> Artificial

<220>
<223> primer

<400> 16
tgaattccat aacgaagaca ccgtcta 27

<210> 17
<211> 24
<212> DNA
<213> Artificial

<220>
<223> primer

<400> 17
caagcagcgg agaccttgca atgc 24

<210> 18
<211> 1656
<212> DNA

<213>  Artificial

<220>
<223>  GalT knock-out

<400>  18
tggcacgata cagtggcatg agatttatcg ctgccaaact gtggacaatg atgtttgaaa      60

cagtctattc atcactggtt ggcaaattct atgtacaggg ctaaaagggc caaactaggc     120

ttaacagcag tgatcgaggt tcttgagcag gatcagcgca agggtaaggt tgcttaggac     180

cgcttcaacc tggtgagtta gacactcaaa ataattacga aacagtgaca tttataagct     240

ttgtgtcgtc actactttga gccttcagag tacatttata ggtggtgact tcgttaatga     300

tgttaaaaat atgaggtgag gacatgtctt cttgtgatta gagtgatcac tttgatcctt     360

ttgcaaacgc tgaaaggagt aagtctgatt gtcaacagaa atgtttttgg ttgcagcctg     420

gctaatatta ttggtctcag ttcaattttc gatggagtgg cgtacaagtg atccagaaag     480

caagaatcat ggatttccta caatttcatt tagattttcg atgttggttg agttatgctg     540

attgatttgg gaaagaggga gcttagcgtt gtatacaggg ttcaaacacc gtaatatgaa     600

gaggggtgtg agaccaccgg gtgtgggatg tacagggcgg caaagaaaca atctaatcat     660

agtggcaatc atatgtttgg tttttatagc gatattcatc ccaccgtttc ttgaatgaat     720

tccataacga agacaccgtc taaagcttca caggttagtg cagaaatgat tggttcgccc     780

tcgctatgcc agtcaggctt actgagttct acttggatcg ttctacttgg atcttttatg     840

gcttcctagc agtcggaggt ttctttctgg tttgaagaaa gccatgtatg gaacgtttac     900

aggttttgga gaagaaagta tcaagctatt tgaaaaaagt cactctggaa acttacagta     960

aagaggaacg ccgtagtcca gggaacacaa caggtgacat tgtttcgctg gaagatgtga    1020

tagatcgcgc ctggtctgcc ggcgccaaag cttgggaaga gctggaaatt gcattcagac    1080

agggagaaca tttttcgaag aaggacaata atgccaatgc aactgcagat ccatgcccag    1140

catcactctt tacaacagga aaggaattgg acaatttagg aagggtcttc ccactgcctt    1200

gtggtctaat gtttggatca gccataactc tcattggaaa gccacgggaa gctcacatgg    1260

agtacaaacc gccaatcgcc agagttgggg aaggtgtctc tccatacgtc atggtgtccc    1320

agttcataat ggagttacag ggcttgaagg tggtaaaagg tgaagatcct cctagaatcc    1380

tccacataaa ccctcgactc cgtggtgact ggagctggaa acccatcatt gagcataata    1440

catgctatcg aaaccagtgg ggcccagctc atcggtgtga aggttggcaa gtacctgaat    1500

acgaagaaac cggtgagtgc tggttccatc acactttatc ttttcatagt gacacggttc    1560

tttttaggtg tactagtgtt gaaagctgtg catgttaaat ggtaacccta atcaatcttc    1620

tcgctaattt tcgcattgca aggtctccgc tgcttg                              1656

<210>  19
<211>  634
<212>  PRT
<213>  Physcomitrella patens

<400>  19

Met Lys Arg Gly Ser Arg Leu Pro Asp Met Ala Cys Thr Gly Arg Gln
1               5                   10                  15

Arg Asn Asp Leu Ile Leu Val Ala Ile Val Cys Leu Phe Phe Met Val
            20                  25                  30

Ile Phe Ile Pro Pro Tyr Leu Gln Met Asn Ser Leu Pro Asp Ile Asp
        35                  40                  45

Ser Pro Asp Ser Asp Lys Lys Ser Ser Ser Tyr Ser Lys Lys Thr Thr
    50                  55                  60

Leu Glu Ala Asn Ser Lys Glu Glu Arg Arg Ser Pro Gly Asn Thr Thr
65                  70                  75                  80

Gly Asp Ile Val Ser Leu Asp Asp Val Ile Asp Arg Ala Trp Ser Ala
                85                  90                  95

Gly Ala Lys Ala Trp Glu Glu Leu Glu Thr Ala Leu Arg Asn Gly Glu
            100                 105                 110

Gly Val Ser Lys Asn Val Ser Asn Ala Thr Ala Asn Ala Asp Pro Cys
        115                 120                 125

Pro Ala Ser Leu Ser Ala Ala Gly Lys Lys Leu Asp Glu Leu Gly Lys
    130                 135                 140

Val Phe Pro Leu Pro Cys Gly Leu Met Phe Gly Ser Ala Ile Thr Leu
145                 150                 155                 160

Ile Gly Lys Pro Arg Glu Ala His Met Glu Tyr Lys Pro Pro Ile Ala
                165                 170                 175

Arg Val Gly Glu Gly Val Ser Pro Tyr Val Met Val Ser Gln Phe Leu
            180                 185                 190

Val Glu Leu Gln Gly Leu Lys Val Val Lys Gly Glu Asp Pro Pro Arg
        195                 200                 205

Ile Leu His Leu Asn Pro Arg Leu Arg Gly Asp Trp Ser Trp Lys Pro
    210                 215                 220

Ile Ile Glu His Asn Thr Cys Tyr Arg Asn Gln Trp Gly Pro Ala His
225                 230                 235                 240

Arg Cys Glu Gly Trp Gln Val Pro Glu Tyr Glu Glu Thr Val Asp Gly
                245                 250                 255

Leu Pro Lys Cys Glu Lys Trp Leu Arg Asp Asp Gly Lys Lys Pro Ala
            260                 265                 270

Ser Thr Gln Lys Ser Trp Trp Leu Gly Arg Leu Val Gly Arg Ser Asp
        275                 280                 285

Lys Glu Thr Leu Glu Trp Glu Tyr Pro Leu Ser Glu Gly Arg Glu Phe
    290                 295                 300

Val Leu Thr Ile Arg Ala Gly Val Glu Gly Phe His Val Thr Ile Asp
305                 310                 315                 320

Gly Arg His Ile Ser Ser Phe Pro Tyr Arg Val Gly Tyr Ala Val Glu
                325                 330                 335

Glu Thr Thr Gly Ile Leu Val Ala Gly Asp Val Asp Val Met Ser Ile
            340                 345                 350

Thr Val Thr Ser Leu Pro Leu Thr His Pro Ser Tyr Tyr Pro Glu Leu
        355                 360                 365

Val Leu Glu Ser Gly Asp Ile Trp Lys Ala Pro Pro Val Pro Ala Thr
    370                 375                 380

Lys Ile Asp Leu Phe Ile Gly Ile Met Ser Ser Ser Asn His Phe Ala
385                 390                 395                 400

Glu Arg Met Ala Val Arg Lys Thr Trp Phe Gln Ser Lys Ala Ile Gln
                405                 410                 415

Ser Ser Gln Ala Val Ala Arg Phe Phe Val Ala Leu His Ala Asn Lys
            420                 425                 430

Asp Ile Asn Met Gln Leu Lys Lys Glu Ala Asp Tyr Tyr Gly Asp Ile
        435                 440                 445

Ile Ile Leu Pro Phe Ile Asp Arg Tyr Asp Ile Val Val Leu Lys Thr
    450                 455                 460

Val Glu Ile Cys Lys Phe Gly Val Gln Asn Val Thr Ala Lys Tyr Ile
465                 470                 475                 480

Met Lys Cys Asp Asp Asp Thr Phe Val Arg Ile Asp Ser Val Leu Glu
                485                 490                 495

Glu Ile Arg Thr Thr Ser Ile Ser Gln Gly Leu Tyr Met Gly Ser Met
            500                 505                 510

Asn Glu Phe His Arg Pro Leu Arg Ser Gly Lys Trp Ala Val Thr Ala
        515                 520                 525

Glu Glu Trp Pro Glu Arg Ile Tyr Pro Ile Tyr Ala Asn Gly Pro Gly
    530                 535                 540

Tyr Ile Leu Ser Glu Asp Ile Val His Phe Ile Val Glu Met Asn Glu
545                 550                 555                 560

Arg Gly Ser Leu Gln Leu Phe Lys Met Glu Asp Val Ser Val Gly Ile
                565                 570                 575

Trp Val Arg Glu Tyr Ala Lys Gln Val Lys His Val Gln Tyr Glu His
            580                 585                 590

Ser Ile Arg Phe Ala Gln Ala Gly Cys Ile Pro Lys Tyr Leu Thr Ala
        595                 600                 605

His Tyr Gln Ser Pro Arg Gln Met Leu Cys Leu Trp Asp Lys Val Leu
    610                 615                 620

Ala His Asp Asp Gly Lys Cys Cys Asn Leu
625                 630

<210>  20
<211>  633
<212>  PRT
<213>  Physcomitrella patens

<400>  20

Met Lys Arg Gly Val Arg Pro Pro Gly Val Gly Cys Thr Gly Arg Gln
1               5                   10                  15

Arg Asn Asn Leu Ile Ile Val Ala Ile Ile Cys Leu Val Phe Ile Ala
            20                  25                  30

Ile Phe Ile Pro Pro Phe Leu Glu Met Asn Ser Leu Pro Asp Ile Asp
        35                  40                  45

Ser Pro Val Leu Glu Lys Lys Val Ser Ser Tyr Leu Lys Lys Val Thr
    50                  55                  60

Leu Glu Thr Tyr Ser Lys Glu Glu Arg Arg Ser Pro Gly Asn Thr Thr
65                  70                  75                  80

Gly Asp Ile Val Ser Leu Glu Asp Val Ile Asp Arg Ala Trp Ser Ala
                85                  90                  95

Gly Ala Lys Ala Trp Glu Glu Leu Glu Ile Ala Phe Arg Gln Gly Glu
            100                 105                 110

His Phe Ser Lys Lys Asp Asn Asn Ala Asn Ala Thr Ala Asp Pro Cys
        115                 120                 125

Pro Ala Ser Leu Phe Thr Thr Gly Lys Glu Leu Asp Asn Leu Gly Arg
    130                 135                 140

Val Phe Pro Leu Pro Cys Gly Leu Met Phe Gly Ser Ala Ile Thr Leu
145                 150                 155                 160

Ile Gly Lys Pro Arg Glu Ala His Met Glu Tyr Lys Pro Pro Ile Ala
                165                 170                 175

Arg Val Gly Glu Gly Val Ser Pro Tyr Val Met Val Ser Gln Phe Ile
            180                 185                 190

Met Glu Leu Gln Gly Leu Lys Val Val Lys Gly Glu Asp Pro Pro Arg
        195                 200                 205

Ile Leu His Ile Asn Pro Arg Leu Arg Gly Asp Trp Ser Trp Lys Pro
    210                 215                 220

Ile Ile Glu His Asn Thr Cys Tyr Arg Asn Gln Trp Gly Pro Ala His
225                 230                 235                 240

Arg Cys Glu Gly Trp Gln Val Pro Glu Tyr Glu Glu Thr Val Asp Gly
                245                 250                 255

Leu Pro Lys Cys Glu Lys Trp Leu Arg Gly Asp Asp Lys Lys Pro Ala
            260                 265                 270

Ser Thr Gln Lys Ser Trp Trp Leu Gly Arg Leu Val Gly His Ser Asp
        275                 280                 285

Lys Glu Thr Leu Glu Trp Glu Tyr Pro Leu Ser Glu Gly Arg Glu Phe
    290                 295                 300

Val Leu Thr Ile Arg Ala Gly Val Glu Gly Phe His Leu Thr Ile Asp
305                 310                 315                 320

Gly Arg His Ile Ser Ser Phe Pro Tyr Arg Ala Gly Tyr Ala Met Glu
                325                 330                 335

Glu Ala Thr Gly Ile Ser Val Ala Gly Asp Val Asp Val Leu Ser Met
            340                 345                 350

Thr Val Thr Ser Leu Pro Leu Thr His Pro Ser Tyr Tyr Pro Glu Leu
        355                 360                 365

Val Leu Asp Ser Gly Asp Ile Trp Lys Ala Pro Pro Leu Pro Thr Gly
    370                 375                 380

Lys Ile Glu Leu Phe Val Gly Ile Met Ser Ser Ser Asn His Phe Ala
385                 390                 395                 400

Glu Arg Met Ala Val Arg Lys Thr Trp Phe Gln Ser Leu Val Ile Gln
                405                 410                 415

Ser Ser Gln Ala Val Ala Arg Phe Phe Val Ala Leu His Ala Asn Lys
            420                 425                 430

Asp Ile Asn Leu Gln Leu Lys Lys Glu Ala Asp Tyr Tyr Gly Asp Met
        435                 440                 445

Ile Ile Leu Pro Phe Ile Asp Arg Tyr Asp Ile Val Val Leu Lys Thr

450                 455                 460

Val Glu Ile Phe Lys Phe Gly Val Gln Asn Val Thr Val Ser His Val
465                 470                 475                 480

Met Lys Cys Asp Asp Asp Thr Phe Val Arg Ile Asp Ser Val Leu Glu
                485                 490                 495

Glu Ile Arg Thr Thr Ser Val Gly Gln Gly Leu Tyr Met Gly Ser Met
            500                 505                 510

Asn Glu Phe His Arg Pro Leu Arg Ser Gly Lys Trp Ala Val Thr Val
        515                 520                 525

Glu Glu Trp Pro Glu Arg Ile Tyr Pro Thr Tyr Ala Asn Gly Pro Gly
    530                 535                 540

Tyr Ile Leu Ser Glu Asp Ile Val His Phe Ile Val Glu Glu Ser Lys
545                 550                 555                 560

Arg Asn Asn Leu Arg Leu Phe Lys Met Glu Asp Val Ser Val Gly Ile
                565                 570                 575

Trp Val Arg Glu Tyr Ala Lys Met Lys Tyr Val Gln Tyr Glu His Ser
            580                 585                 590

Val Arg Phe Ala Gln Ala Gly Cys Ile Pro Asn Tyr Leu Thr Ala His
        595                 600                 605

Tyr Gln Ser Pro Arg Gln Met Leu Cys Leu Trp Asp Lys Val Leu Ala
    610                 615                 620

Thr Asn Asp Gly Lys Cys Cys Thr Leu
625                 630

<210>  21
<211>  422
<212>  PRT
<213>  homo sapiens

<400>  21

Met Leu Gln Trp Arg Arg Arg His Cys Cys Phe Ala Lys Met Thr Trp
1               5                   10                  15

Asn Ala Lys Arg Ser Leu Phe Arg Thr His Leu Ile Gly Val Leu Ser
            20                  25                  30

Leu Val Phe Leu Phe Ala Met Phe Leu Phe Phe Asn His His Asp Trp
        35                  40                  45

Leu Pro Gly Arg Ala Gly Phe Lys Glu Asn Pro Val Thr Tyr Thr Phe
    50                  55                  60

Arg Gly Phe Arg Ser Thr Lys Ser Glu Thr Asn His Ser Ser Leu Arg
65                  70                  75                  80

Asn Ile Trp Lys Glu Thr Val Pro Gln Thr Leu Arg Pro Gln Thr Ala
                85                  90                  95

Thr Asn Ser Asn Asn Thr Asp Leu Ser Pro Gln Gly Val Thr Gly Leu
            100                 105                 110

Glu Asn Thr Leu Ser Ala Asn Gly Ser Ile Tyr Asn Glu Lys Gly Thr
        115                 120                 125

Gly His Pro Asn Ser Tyr His Phe Lys Tyr Ile Ile Asn Glu Pro Glu
    130                 135                 140

Lys Cys Gln Glu Lys Ser Pro Phe Leu Ile Leu Leu Ile Ala Ala Glu
145                 150                 155                 160

Pro Gly Gln Ile Glu Ala Arg Arg Ala Ile Arg Gln Thr Trp Gly Asn
                165                 170                 175

Glu Ser Leu Ala Pro Gly Ile Gln Ile Thr Arg Ile Phe Leu Leu Gly
            180                 185                 190

Leu Ser Ile Lys Leu Asn Gly Tyr Leu Gln Arg Ala Ile Leu Glu Glu
        195                 200                 205

Ser Arg Gln Tyr His Asp Ile Ile Gln Gln Glu Tyr Leu Asp Thr Tyr
    210                 215                 220

Tyr Asn Leu Thr Ile Lys Thr Leu Met Gly Met Asn Trp Val Ala Thr
225                 230                 235                 240

Tyr Cys Pro His Ile Pro Tyr Val Met Lys Thr Asp Ser Asp Met Phe
                245                 250                 255

Val Asn Thr Glu Tyr Leu Ile Asn Lys Leu Leu Lys Pro Asp Leu Pro
            260                 265                 270

Pro Arg His Asn Tyr Phe Thr Gly Tyr Leu Met Arg Gly Tyr Ala Pro
        275                 280                 285

Asn Arg Asn Lys Asp Ser Lys Trp Tyr Met Pro Pro Asp Leu Tyr Pro
    290                 295                 300

Ser Glu Arg Tyr Pro Val Phe Cys Ser Gly Thr Gly Tyr Val Phe Ser
305                 310                 315                 320

Gly Asp Leu Ala Glu Lys Ile Phe Lys Val Ser Leu Gly Ile Arg Arg
                325                 330                 335

Leu His Leu Glu Asp Val Tyr Val Gly Ile Cys Leu Ala Lys Leu Arg
            340                 345                 350

Ile Asp Pro Val Pro Pro Pro Asn Glu Phe Val Phe Asn His Trp Arg
        355                 360                 365

Val Ser Tyr Ser Ser Cys Lys Tyr Ser His Leu Ile Thr Ser His Gln
    370                 375                 380

Phe Gln Pro Ser Glu Leu Ile Lys Tyr Trp Asn His Leu Gln Gln Asn
385                 390                 395                 400

Lys His Asn Ala Cys Ala Asn Ala Ala Lys Glu Lys Ala Gly Arg Tyr
                405                 410                 415

Arg His Arg Lys Leu His
            420

<210>  22
<211>  621
<212>  PRT
<213>  Oryza sativa

<400>  22

Met Trp Val Thr Lys Arg Leu Gly Ile Thr Val Leu Ile Val Leu Phe
1               5                   10                  15

Pro Leu Leu Ile Val His His Leu Ile Val Asn Ser Pro Val Ser Gly
            20                  25                  30

Pro Ser Arg Tyr Gln Val Ile His Ser Asn Leu Leu Gly Trp Leu Ser
        35                  40                  45

Asp Ser Leu Gly Asn Ser Val Ala Gln Asn Pro Asp Asn Thr Pro Val
    50                  55                  60

Glu Val Ile Pro Ala Asp Ala Ser Ala Ser Asn Ser Ser Asp Ser Gly
65                  70                  75                  80

Asn Ser Ser Leu Glu Gly Phe Gln Trp Leu Asn Thr Trp Asn His Met
                85                  90                  95

Lys Gln Leu Thr Asn Ile Ser Asp Gly Leu Pro His Ala Asn Glu Ala
            100                 105                 110

Ile Asp Asn Ala Arg Thr Ala Trp Glu Asn Leu Thr Ile Ser Val His
        115                 120                 125

Asn Ser Thr Ser Lys Gln Ile Lys Lys Glu Arg Gln Cys Pro Tyr Ser
    130                 135                 140

Ile His Arg Met Asn Ala Ser Lys Pro Asp Thr Gly Asp Phe Thr Ile
145                 150                 155                 160

Asp Ile Pro Cys Gly Leu Ile Val Gly Ser Ser Val Thr Ile Ile Gly

                165                 170                 175

Thr Pro Gly Ser Leu Ser Gly Asn Phe Arg Ile Asp Leu Val Gly Thr
            180                 185                 190

Glu Leu Pro Gly Gly Ser Gly Lys Pro Ile Val Leu His Tyr Asp Val
        195                 200                 205

Arg Leu Thr Ser Asp Glu Leu Thr Gly Gly Pro Val Ile Val Gln Asn
    210                 215                 220

Ala Phe Thr Ala Ser Asn Gly Trp Gly Tyr Glu Asp Arg Cys Pro Cys
225                 230                 235                 240

Ser Asn Cys Asn Asn Ala Thr Gln Val Asp Asp Leu Glu Arg Cys Asn
                245                 250                 255

Ser Met Val Gly Arg Glu Glu Lys Arg Ala Ile Asn Ser Lys Gln His
            260                 265                 270

Leu Asn Ala Lys Lys Asp Glu His Pro Ser Thr Tyr Phe Pro Phe Lys
        275                 280                 285

Gln Gly His Leu Ala Ile Ser Thr Leu Arg Ile Gly Leu Glu Gly Ile
    290                 295                 300

His Met Thr Val Asp Gly Lys His Val Thr Ser Phe Pro Tyr Lys Ala
305                 310                 315                 320

Gly Leu Glu Ala Trp Phe Val Thr Glu Val Gly Val Ser Gly Asp Phe
                325                 330                 335

Lys Leu Val Ser Ala Ile Ala Ser Gly Leu Pro Thr Ser Glu Asp Leu
            340                 345                 350

Glu Asn Ser Phe Asp Leu Ala Met Leu Lys Ser Ser Pro Ile Pro Glu
        355                 360                 365

Gly Lys Asp Val Asp Leu Leu Ile Gly Ile Phe Ser Thr Ala Asn Asn
    370                 375                 380

Phe Lys Arg Arg Met Ala Ile Arg Arg Thr Trp Met Gln Tyr Asp Ala
385                 390                 395                 400

Val Arg Glu Gly Ala Val Val Val Arg Phe Phe Val Gly Leu His Thr
                405                 410                 415

Asn Leu Ile Val Asn Lys Glu Leu Trp Asn Glu Ala Arg Thr Tyr Gly
            420                 425                 430

Asp Ile Gln Val Leu Pro Phe Val Asp Tyr Tyr Ser Leu Ile Thr Trp
        435                 440                 445

Lys Thr Leu Ala Ile Cys Ile Tyr Gly Thr Gly Ala Val Ser Ala Lys
    450                 455                 460

Tyr Leu Met Lys Thr Asp Asp Asp Ala Phe Val Arg Val Asp Glu Ile
465                 470                 475                 480

His Ser Ser Val Lys Gln Leu Asn Val Ser His Gly Leu Leu Tyr Gly
                485                 490                 495

Arg Ile Asn Ser Asp Ser Gly Pro His Arg Asn Pro Glu Ser Lys Trp
            500                 505                 510

Tyr Ile Ser Pro Glu Glu Trp Pro Glu Glu Lys Tyr Pro Pro Trp Ala
        515                 520                 525

His Gly Pro Gly Tyr Val Val Ser Gln Asp Ile Ala Lys Glu Ile Asn
    530                 535                 540

Ser Trp Tyr Glu Thr Ser His Leu Lys Met Phe Lys Leu Glu Asp Val
545                 550                 555                 560

Ala Met Gly Ile Trp Ile Ala Glu Met Lys Lys Gly Gly Leu Pro Val
                565                 570                 575

Gln Tyr Lys Thr Asp Glu Arg Ile Asn Ser Asp Gly Cys Asn Asp Gly
            580                 585                 590

Cys Ile Val Ala His Tyr Gln Glu Pro Arg His Met Leu Cys Met Trp
        595                 600                 605

Glu Lys Leu Leu Arg Thr Asn Gln Ala Thr Cys Cys Asn
    610                 615                 620

<210>  23
<211>  643
<212>  PRT
<213>  Arabidopsis thaliana

<400>  23

Met Lys Arg Phe Tyr Gly Gly Leu Leu Val Val Ser Met Cys Met Phe
1               5                   10                  15

Leu Thr Val Tyr Arg Tyr Val Asp Leu Asn Thr Pro Val Glu Lys Pro
            20                  25                  30

Tyr Ile Thr Ala Ala Ala Ser Val Val Val Thr Pro Asn Thr Thr Leu
        35                  40                  45

Pro Met Glu Trp Leu Arg Ile Thr Leu Pro Asp Phe Met Lys Glu Ala
    50                  55                  60

Arg Asn Thr Gln Glu Ala Ile Ser Gly Asp Asp Ile Ala Val Val Ser
65                  70                  75                  80

Gly Leu Phe Val Glu Gln Asn Val Ser Lys Glu Glu Arg Glu Pro Leu
                85                  90                  95

Leu Thr Trp Asn Arg Leu Glu Ser Leu Val Asp Asn Ala Gln Ser Leu
            100                 105                 110

Val Asn Gly Val Asp Ala Ile Lys Glu Ala Gly Ile Val Trp Glu Ser
        115                 120                 125

Leu Val Ser Ala Val Glu Ala Lys Lys Leu Val Asp Val Asn Glu Asn
    130                 135                 140

Gln Thr Arg Lys Gly Lys Glu Glu Leu Cys Pro Gln Phe Leu Ser Lys
145                 150                 155                 160

Met Asn Ala Thr Glu Ala Asp Gly Ser Ser Leu Lys Leu Gln Ile Pro
                165                 170                 175

Cys Gly Leu Thr Gln Gly Ser Ser Ile Thr Val Ile Gly Ile Pro Asp
            180                 185                 190

Gly Leu Val Gly Ser Phe Arg Ile Asp Leu Thr Gly Gln Pro Leu Pro
        195                 200                 205

Gly Glu Pro Asp Pro Pro Ile Ile Val His Tyr Asn Val Arg Leu Leu
    210                 215                 220

Gly Asp Lys Ser Thr Glu Asp Pro Val Ile Val Gln Asn Ser Trp Thr
225                 230                 235                 240

Ala Ser Gln Asp Trp Gly Ala Glu Glu Arg Cys Pro Lys Phe Asp Pro
                245                 250                 255

Asp Met Asn Lys Lys Val Asp Asp Leu Asp Glu Cys Asn Lys Met Val
            260                 265                 270

Gly Gly Glu Ile Asn Arg Thr Ser Ser Thr Ser Leu Gln Ser Asn Thr
        275                 280                 285

Ser Arg Gly Val Pro Val Ala Arg Glu Ala Ser Lys His Glu Lys Tyr
    290                 295                 300

Phe Pro Phe Lys Gln Gly Phe Leu Ser Val Ala Thr Leu Arg Val Gly
305                 310                 315                 320

Thr Glu Gly Met Gln Met Thr Val Asp Gly Lys His Ile Thr Ser Phe
                325                 330                 335

Ala Phe Arg Asp Thr Leu Glu Pro Trp Leu Val Ser Glu Ile Arg Ile
            340                 345                 350

Thr Gly Asp Phe Arg Leu Ile Ser Ile Leu Ala Ser Gly Leu Pro Thr
        355                 360                 365

Ser Glu Glu Ser Glu His Val Val Asp Leu Glu Ala Leu Lys Ser Pro
    370                 375                 380

Thr Leu Ser Pro Leu Arg Pro Leu Asp Leu Val Ile Gly Val Phe Ser
385                 390                 395                 400

Thr Ala Asn Asn Phe Lys Arg Arg Met Ala Val Arg Arg Thr Trp Met
                405                 410                 415

Gln Tyr Asp Asp Val Arg Ser Gly Arg Val Ala Val Arg Phe Phe Val
            420                 425                 430

Gly Leu His Lys Ser Pro Leu Val Asn Leu Glu Leu Trp Asn Glu Ala
        435                 440                 445

Arg Thr Tyr Gly Asp Val Gln Leu Met Pro Phe Val Asp Tyr Tyr Ser
    450                 455                 460

Leu Ile Ser Trp Lys Thr Leu Ala Ile Cys Ile Phe Gly Thr Glu Val
465                 470                 475                 480

Asp Ser Ala Lys Phe Ile Met Lys Thr Asp Asp Asp Ala Phe Val Arg
                485                 490                 495

Val Asp Glu Val Leu Leu Ser Leu Ser Met Thr Asn Asn Thr Arg Gly
            500                 505                 510

Leu Ile Tyr Gly Leu Ile Asn Ser Asp Ser Gln Pro Ile Arg Asn Pro
        515                 520                 525

Asp Ser Lys Trp Tyr Ile Ser Tyr Glu Glu Trp Pro Glu Glu Lys Tyr
    530                 535                 540

Pro Pro Trp Ala His Gly Pro Gly Tyr Ile Val Ser Arg Asp Ile Ala
545                 550                 555                 560

Glu Ser Val Gly Lys Leu Phe Lys Glu Gly Asn Leu Lys Met Phe Lys
                565                 570                 575

Leu Glu Asp Val Ala Met Gly Ile Trp Ile Ala Glu Leu Thr Lys His
            580                 585                 590

Gly Leu Glu Pro His Tyr Glu Asn Asp Gly Arg Ile Ile Ser Asp Gly
        595                 600                 605

Cys Lys Asp Gly Tyr Val Val Ala His Tyr Gln Ser Pro Ala Glu Met
    610                 615                 620

Thr Cys Leu Trp Arg Lys Tyr Gln Glu Thr Lys Arg Ser Leu Cys Cys
625                 630                 635                 640

Arg Glu Trp